# EUROPEAN PATENT SPECIFICATION

(11) **EP 2 223 114 B1**
(45) Date of publication and mention of the grant of the patent: **17.08.2016**
(21) Application number: 08865264.9
(22) Date of filing: 23.12.2008
(51) Int. Cl.: G01N 33/536, G01N 33/564

(54) **CALIBRATOR FOR IMMUNOASSAYS**
KALIBRIEREINRICHTUNG FÜR IMMUNTESTS
CALIBREUR POUR IMMUNOESSAI

(30) Priority: 24.12.2007 GB 0725239; 26.12.2007 US 16689 P
(43) Date of publication of application: 01.09.2010
(73) Proprietor: OncImmune Limited, Nottingham NG5 1PB (GB)
(72) Inventor: ROBERTSON, John, Forsyth, Russell, Nottingham Nottinghamshire NG9 6AF (GB); MURRAY, Andrea, Leicester Leicestershire LE12 9ND (GB); CHAPMAN, Caroline, Leicester Leicestershire LE12 5PE (GB); BARNES, Anthony, Dunwoody GA 30338 (US)
(74) Representative: Boult Wade Tennant
(86) International application number: PCT/GB2008/004260
(87) International publication number: WO 2009/081165

(56) References cited:
- EP-A- 0 155 141
- WO-A-2004/044590
- WO-A-2006/126008
- WO-A-2008/032084
- GB-A- 2 426 581
- RASMUSSEN N ET AL: "AN ELISA FOR THE DETECTION OF ANTI-NEUTROPHIL CYTOPLASM ANTIBODIES ANCA" JOURNAL OF IMMUNOLOGICAL METHODS, ELSEVIER SCIENCE PUBLISHERS B.V.,AMSTERDAM, NL, vol. 127, no. 1, 1 January 1990 (1990-01-01), pages 139-146, XP002412156 ISSN: 0022-1759
- SCHJETLEIN R ET AL: "Choice of standard plasma for diagnosis and quantitation of lupus anticoagulants" THROMBOSIS RESEARCH, TARRYTOWN, NY, US, vol. 72, no. 4, 15 November 1993 (1993-11-15), pages 287-294, XP000197185 ISSN: 0049-3848
- N G RAINOV ET AL: 'ABSCENCE OF P53 AUTOANTIBODIES IN SERA FROM GLIOMA PATIENTS' CLIN. CANCER RES. vol. 1, 01 July 1995, pages 775 - 781, XP055212836

## Description

### Field of the invention

The invention generally relates to the field of immunoassays. In particular, the invention relates to use of a calibrator material to calibrate immunoassays for autoantibodies.

### Background to the invention

Day to day variation is inherent in any immunoassay. This variation can be due to a number of varying factors including ambient conditions, ageing of the measuring instrument or reagents, batch changes in reagents and biological variation. In longitudinal studies when one needs to compare a test result on one day with another measured on a different day, it is necessary to be able to adjust for this variation. Calibration of the assay makes this possible and can also alert the operator to problems with the output or day to day drift in the instrument.

In general, calibrating an immunoassay designed to measure an antigen in serum is relatively straight forward, since recombinant or synthetic forms of the antigen can often be produced and easily quantified. Hence a highly characterised and clearly defined calibrator material can be made available.

For assays designed to measure the level of human autoantibodies in a patient test sample, the identification of a suitable calibrator material is hampered by the diverse specificity of antibodies being measured and the polyclonality of the response. The present inventors have investigated the use of mouse monoclonal antibodies as calibrators for autoantibody assays. However, these require a different reporter system to that used to detect human autoantibodies so one can never guarantee that variation detected is a true representation of variation inherent in the autoantibody assay. In addition the monoclonal antibodies have highly defined specificity and lack the promiscuity demonstrated by human polyclonal responses. This means that subtle changes in capture antigen structure resulting in assay variation may go undetected. If one were to engineer a humanised antibody for use as a calibrator material this would employ the same reporter system as the autoantibody assay, but would still exhibit the same problems of monoclonality as its murine counterpart.

The inventors therefore had to seek a new source of calibration material which would provide a long term source of calibration both in terms of having sufficient volume and also its ability to be stored for a prolonged period of time.

### Summary of the invention

In a first aspect the invention relates to use of a calibrator material comprising mammalian, and especially human, bodily fluid to calibrate an immunoassay for detection of autoantibodies, wherein said calibrator material comprises a drainage fluid, exudate or transudate.

In one embodiment the calibrator material comprises human bodily fluid.

The calibrator material comprises a drainage fluid, exudate or transudate. This material preferably does not contain any blood products selected from the group consisting of serum, whole blood and plasma.

In one embodiment the calibrator material comprises bodily fluid collected from one or more subjects with cancer.

In another embodiment the calibration material comprises bodily fluid collected from a body cavity or space in which a tumour is or was present or with which a tumour is or was associated.

In certain non-limiting embodiments the calibration material may comprise pleural fluid or ascites fluid collected from one or more human cancer patients.

In one embodiment the calibration material contains native human autoantibodies immunologically specific for a tumour marker protein and the immunoassay to be calibrated is an immunoassay for detection of native human autoantibodies immunologically specific for a tumour marker protein.

In a second aspect the invention provides a method of calibrating an immunoassay for detection of autoantibodies which comprises:
(a) contacting each of a plurality of different dilutions of a calibration material comprising a mammalian bodily fluid with an antigen specific for the autoantibody to be detected in the immunoassay, wherein said bodily fluid is known to contain autoantibodies immunologically specific for the antigen, and wherein the calibrator material comprises a drainage fluid, exudate or transudate;
(b) detecting the amount of specific binding between said antigen and autoantibody present in the calibration material; and
(c) plotting or calculating a curve of the amount of said specific binding versus the dilution of the calibration material for each dilution of calibration material used in step (a), thereby calibrating an immunoassay using said antigen for detection of said autoantibody.

In one embodiment the calibrator material comprises human bodily fluid.

The calibrator material comprises a drainage fluid, exudate or transudate. This material preferably does not contain any blood products selected from the group consisting of serum, whole blood and plasma.

In one embodiment the calibrator material comprises bodily fluid collected from one or more subjects with cancer.

In another embodiment the calibration material comprises bodily fluid collected from a body cavity or space in which a tumour is or was present or with which a tumour is or was associated.

In one embodiment the calibrator material comprises mammalian, and in particular human, bodily fluid collected from a body cavity or space in which a tumour is or was present or with which a tumour is or was associated.

In certain non-limiting embodiments the calibration material may comprise pleural fluid or ascites fluid collected from one or more human cancer patients.

In one embodiment the calibration material contains native human autoantibodies immunologically specific for a tumour marker protein and the immunoassay to be calibrated is an immunoassay for detection of native human autoantibodies immunologically specific for a tumour marker protein.

Therefore, in one specific non-limiting embodiment the invention provides a method of calibrating an immunoassay for detection of anti-tumour marker autoantibodies which comprises:
(a) contacting each of a plurality of different dilutions of a calibration material comprising pleural fluid or ascites fluid isolated from one or more cancer patients with a tumour marker antigen specific for the anti-tumour marker autoantibody, wherein said pleural fluid or ascites fluid is known to contain autoantibodies immunologically specific for said antigen, and wherein the calibrator material comprises a drainage fluid, exudate or transudate;
(b) detecting the amount of specific binding between said antigen and autoantibody present in the calibration material; and
(c) plotting or calculating a curve of the amount of said specific binding versus the dilution of the calibration material for each dilution of calibration material used in step (a), thereby calibrating an immunoassay using said antigen for detection of said autoantibody.

In a third aspect the invention provides an immunoassay kit for detection of autoantibodies, said kit comprising a set of calibration standards for use in calibrating an immunoassay for detection of autoantibodies, wherein each calibration standard in said set comprises a different dilution of a mammalian bodily fluid, said mammalian bodily fluid being known to contain native human autoantibodies, and wherein the mammalian bodily fluid is a drainage fluid, exudate or transudate, and an immunoassay reagent comprising an antigen immunologically specific for said autoantibodies.

In one embodiment the mammalian bodily fluid is a human bodily fluid.

In one embodiment the mammalian bodily fluid does not comprise any blood products selected from the group consisting of serum, whole blood and plasma.

The mammalian bodily fluid is a drainage fluid, exudate or transudate.

In one embodiment the mammalian bodily fluid is fluid collected from one or more subjects with cancer.

In one embodiment the mammalian bodily fluid is bodily fluid collected from a body cavity or space in which a tumour is or was present or with which a tumour is or was associated.

In one embodiment the mammalian bodily fluid comprises pleural fluid collected from one or more subjects with cancer, such as human cancer patients.

In one embodiment the mammalian bodily fluid comprises ascites fluid collected from one or more subjects with cancer, such as human cancer patients.

### Brief description of the drawings

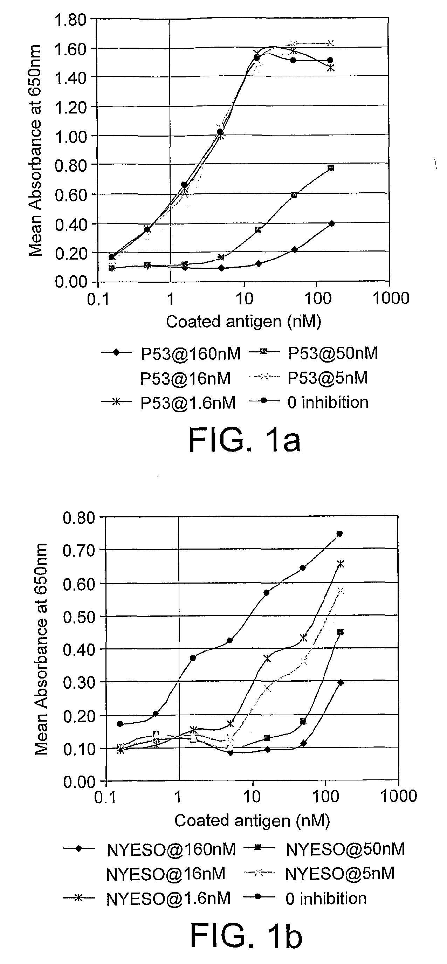

Figure 1: Antigen specific inhibition of human autoantibodies in pleural fluids from advanced breast cancer patients.

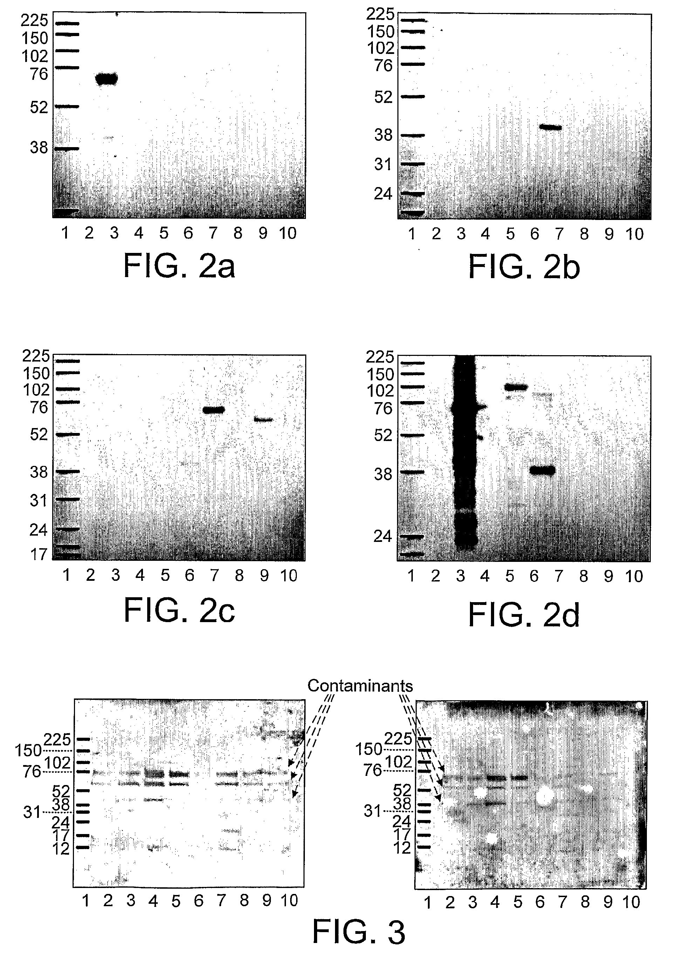

Figure 2: Specificity of pleural fluids for recombinant cancer-associated antigens demonstrated by Western Blotting. (a) fluid B3280 specific for p53, (b) fluid B1564 specific for NY-ESO-1, (c) fluid PL-061 specific for BGU4-5 and Annexin 1, (d) fluid B3084 specific for p53, CAGE and NY-ESO-1. Lane 1 = molecular weight markers, lane 2 = VOL, lane 3 = p53, lane 4 = c-myc, lane 5 = CAGE, lane 6 = NY-ESO-1, lane 7 = GBU4-5, lane 8 = IKBKE, lane 9 = Annexin 1, lane 10 = Annexin 2.
Figure 3: Binding of serum to contaminating bacterial proteins in recombinant proteins demonstrated by Western Blotting. (a), (b). Lane 1 = molecular weight markers, lane 2 = Annexin Xla, lane 3 = BRCA2, lane 4 = c-myc, lane 5 = ECD6, lane 6 = IKBKE, lane 7 = NY-ESO-1, lane 8 = p53, lane 9 = PSA, lane 10 = VOL.

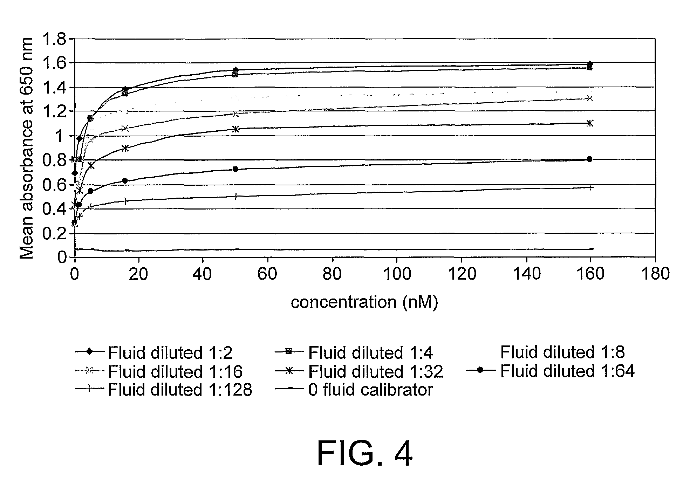

Figure 4: This figure depicts the patient fluid dilutions against the titrating nM concentration of NYESO coated on the plate.

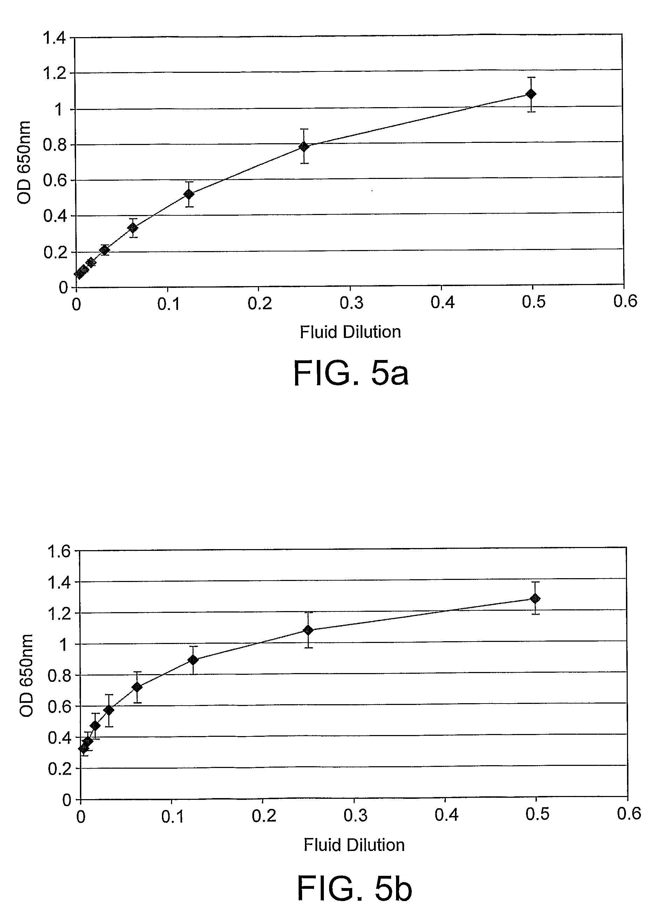

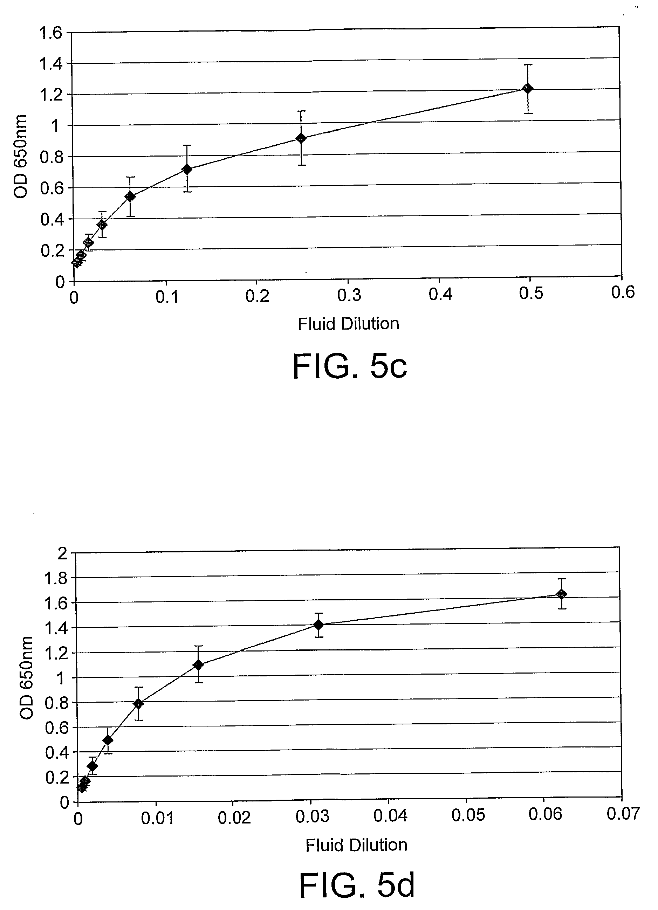

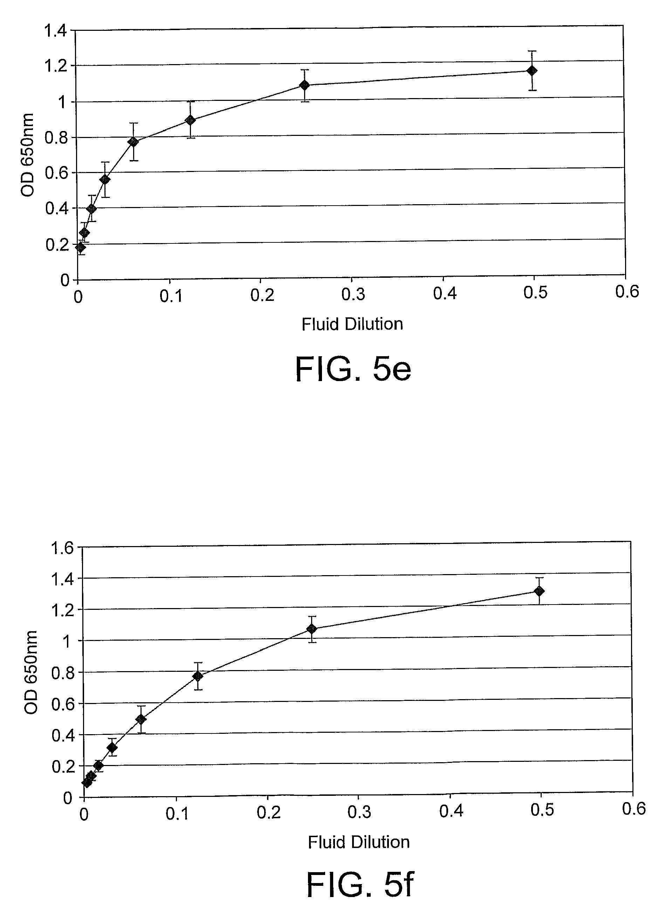

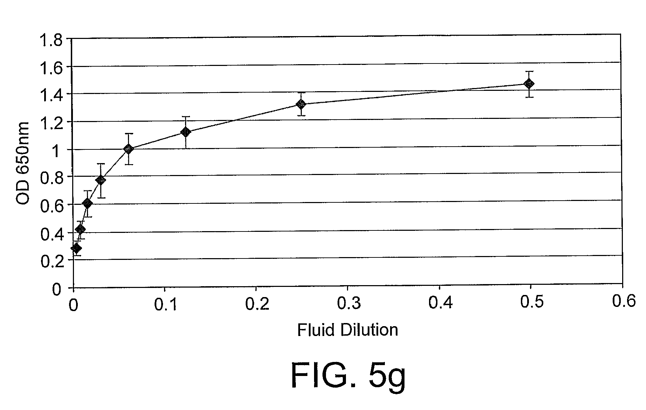

Figure 5: Reproducibility of calibration curves produced using drainage fluids. The curve represents the mean of ten runs with inter-assay variation represented by the standard deviation shown as error bars. Reactivity to p53 (a), c-myc (b), ECD6 (c), NYESO (d), BRCA2 (e) PSA (f) and Annexin Xla (g) are shown.

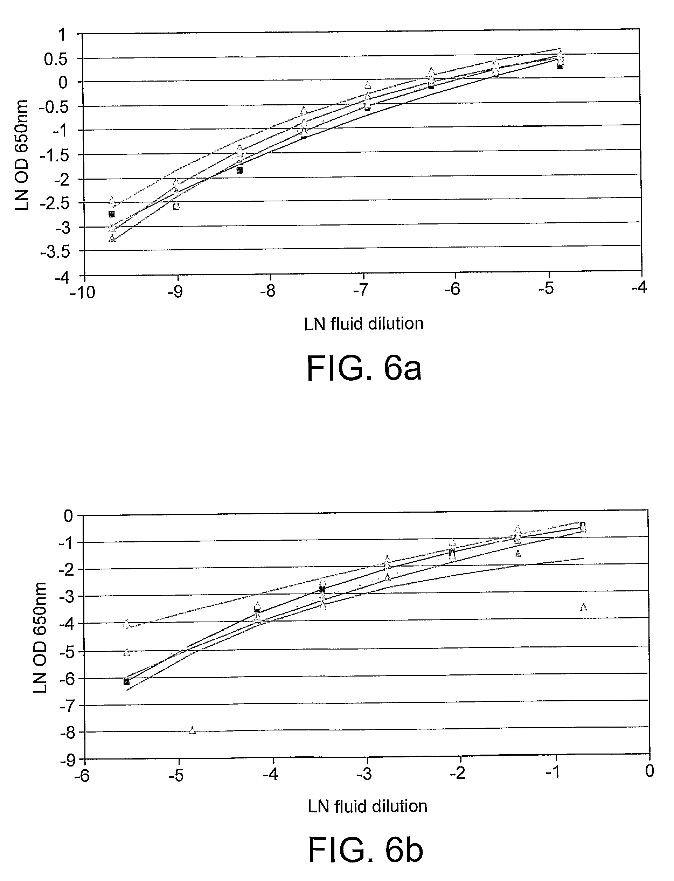

Figure 6: Patient pleural fluid pool C3/C4 (a) and patient pleural fluid pool B3255/B3258 (b) reactivity against 160nM of NYESO in 5 runs where the log fluid dilution is plotted against the logged OD. Data is corrected for non-specific binding by subtracting the signal obtained from binding to the negative control antigen, VOL.

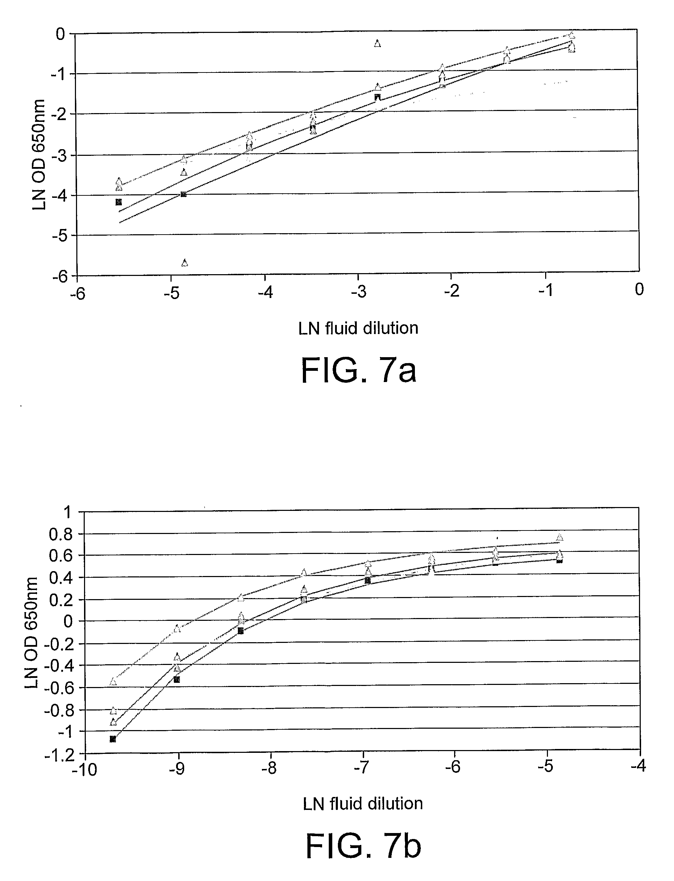

Figure 7: Patient pleural fluid pool B3255/B3258 (a) and patient pleural fluid pool C3/C4 (b) reactivity against 160nM of p53 in 5 runs where the logged fluid dilution is plotted against the logged optical density. Data is corrected for non-specific binding by subtracting the signal obtained from binding to the negative control antigen, VOL.

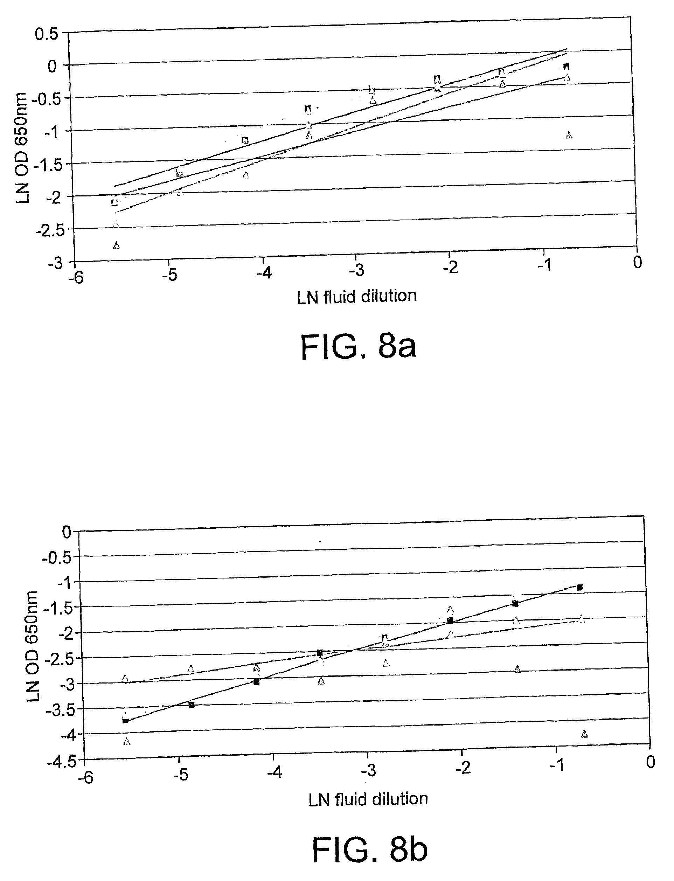

Figure 8: Patient pleural fluid pool B3255/B3258 (a) and patient pleural fluid pool C3/C4 (b) reactivity against 160nM of BRCA2 in 5 runs where the logged fluid dilution is plotted against the logged OD. Data is corrected for non-specific binding by subtracting the signal obtained from binding to the negative control antigen, VOL.

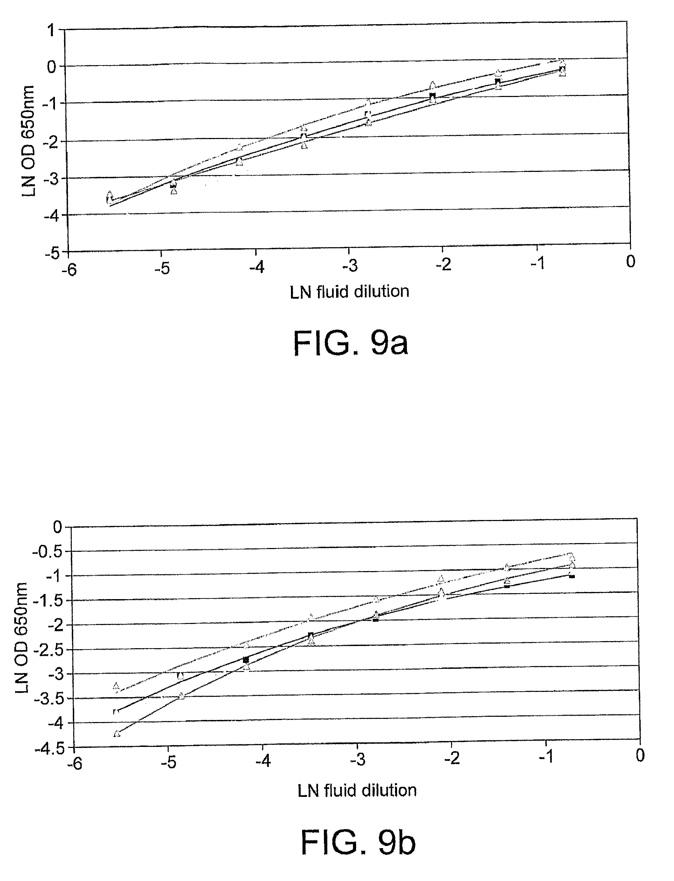

Figure 9: Patient pleural fluid pool B3255/B3258 (a) and patient pleural fluid pool C3/C4 (b) reactivity against 160nM of c-myc in 5 runs where the logged fluid dilution is plotted against the logged OD. Data is corrected for non-specific binding by subtracting the signal obtained from binding to the negative control antigen, VOL.

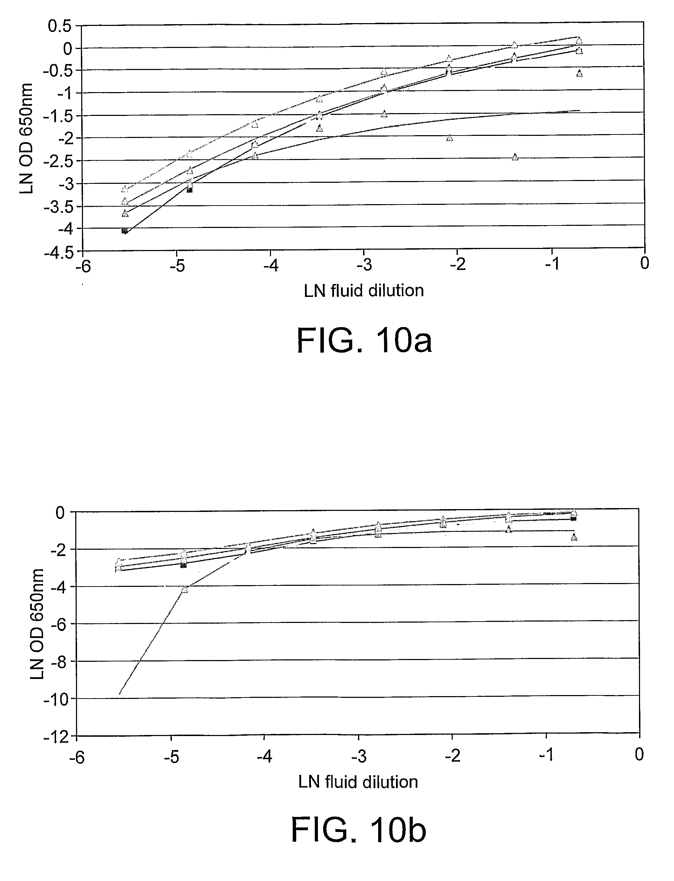

Figure 10: Patient pleural fluid pool B3255/B3258 (a) and patient pleural fluid pool C3/C4 (b) reactivity against 160nM of PSA in 5 runs where the logged fluid dilution is plotted against the logged OD. Data is corrected for non-specific binding by subtracting the signal obtained from binding to the negative control antigen, VOL.

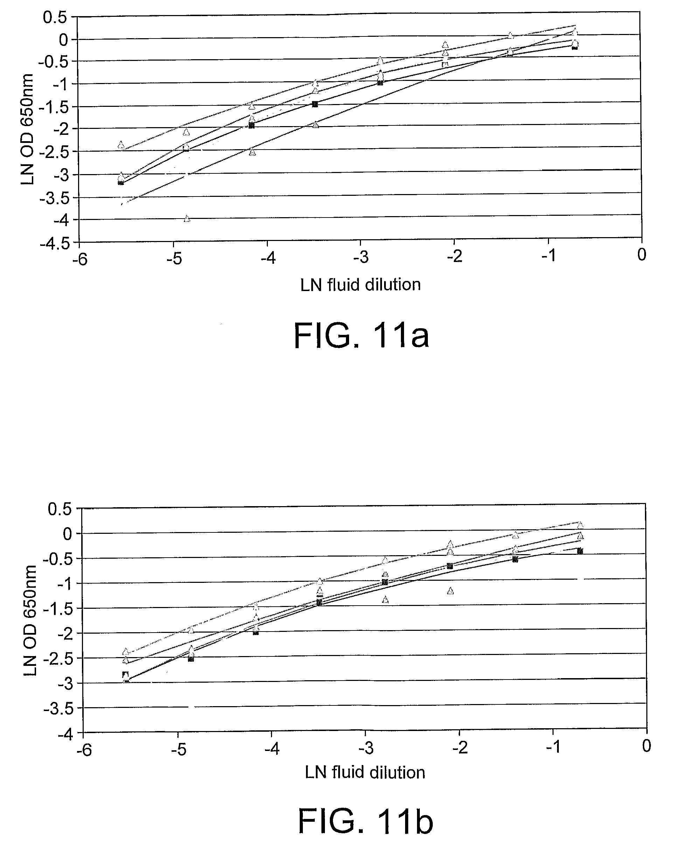

Figure 11: Patient pleural fluid pool B3258/B3255 (a) and patient pleural fluid pool C3/C4 (b) against 160nM of ECD6 in 5 runs where the logged fluid dilution is plotted against the logged OD. Data is corrected for non-specific binding by subtracting the signal obtained from binding to the negative control antigen, VOL.

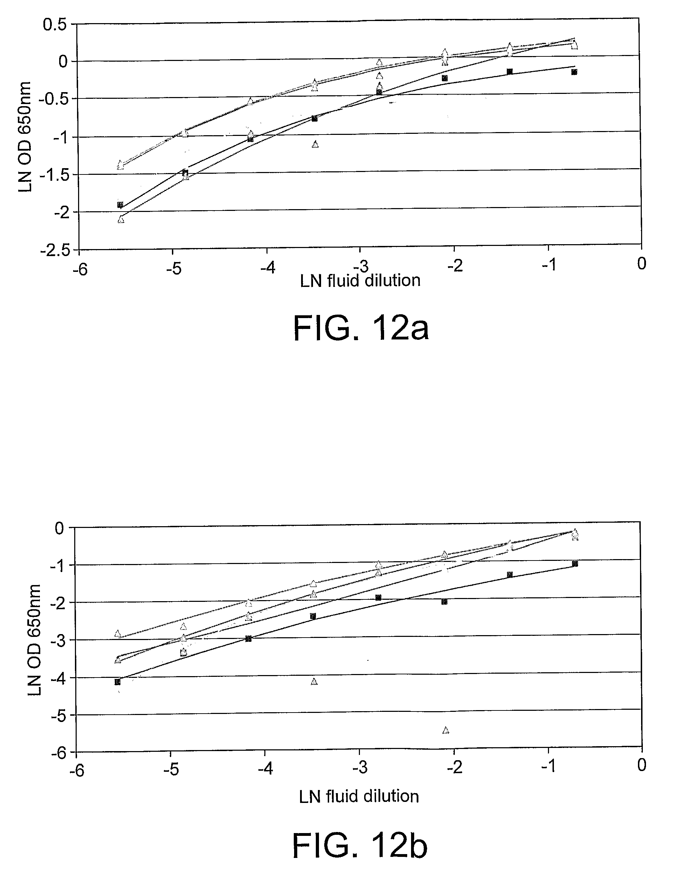

Figure 12: Patient pleural fluid pool B3255/B3258 (a) and patient pleural fluid pool C3/C4 (b) reactivity against 160nM of Annexin Xla in 5 runs where the logged fluid dilution is plotted against the logged OD. Data is corrected for non-specific binding by subtracting the signal obtained from binding to the negative control antigen, VOL.

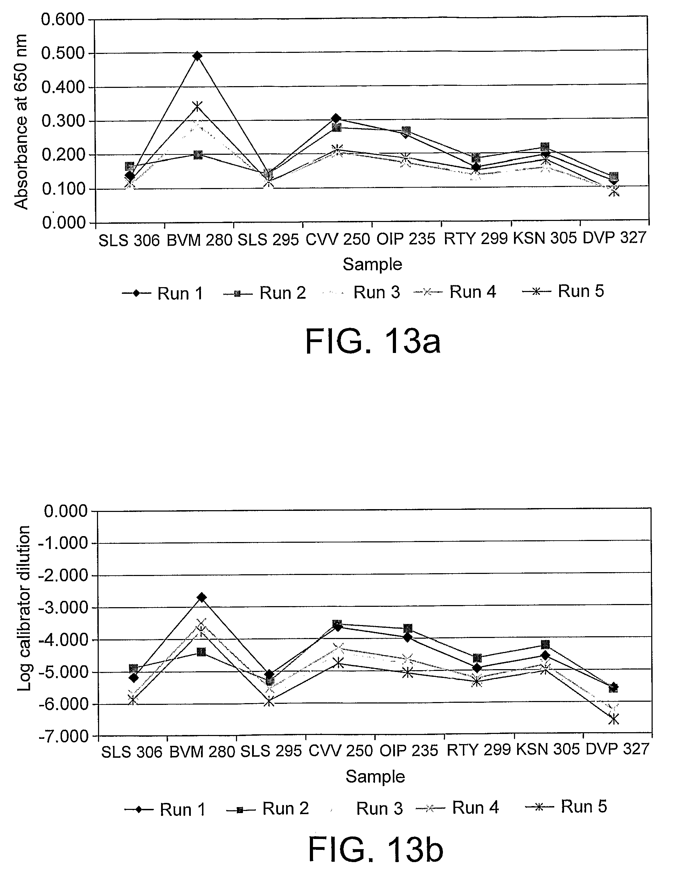

Figure 13: Effect of calibration on the reproducibility of control samples. Autoantibodies against p53 were measured in 8 control sera on 5 separate occasions. The raw OD values are shown in (a). A calibrator curve was run concurrently and this was used to extrapolate values for control samples (b).

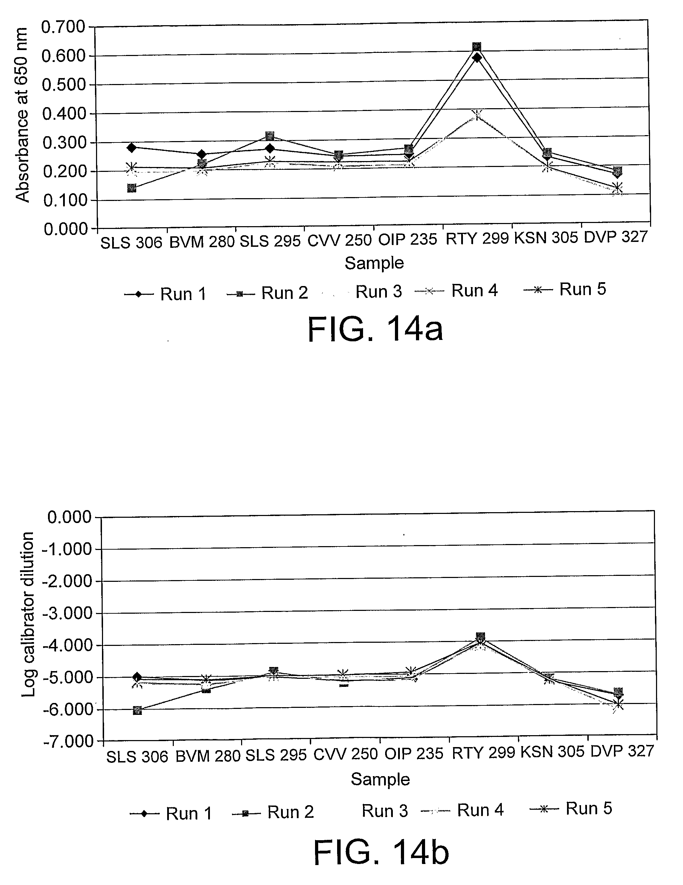

Figure 14: Effect of calibration on the reproducibility of control samples. Autoantibodies against c-myc were measured in 8 control sera on 5 separate occasions. The raw OD values are shown in (a). A calibrator curve was run concurrently and this was used to extrapolate values for control samples (b).

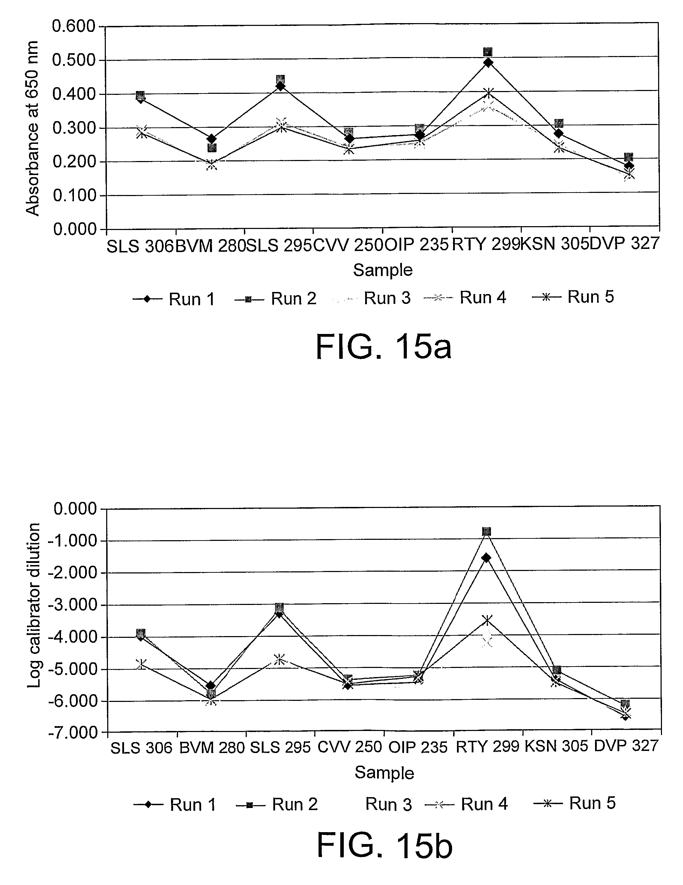

Figure 15: Effect of calibration on the reproducibility of control samples. Autoantibodies against ECD6 were measured in 8 control sera on 5 separate occasions. The raw OD values are shown in (a). A calibrator curve was run concurrently and this was used to extrapolate values for control samples (b).

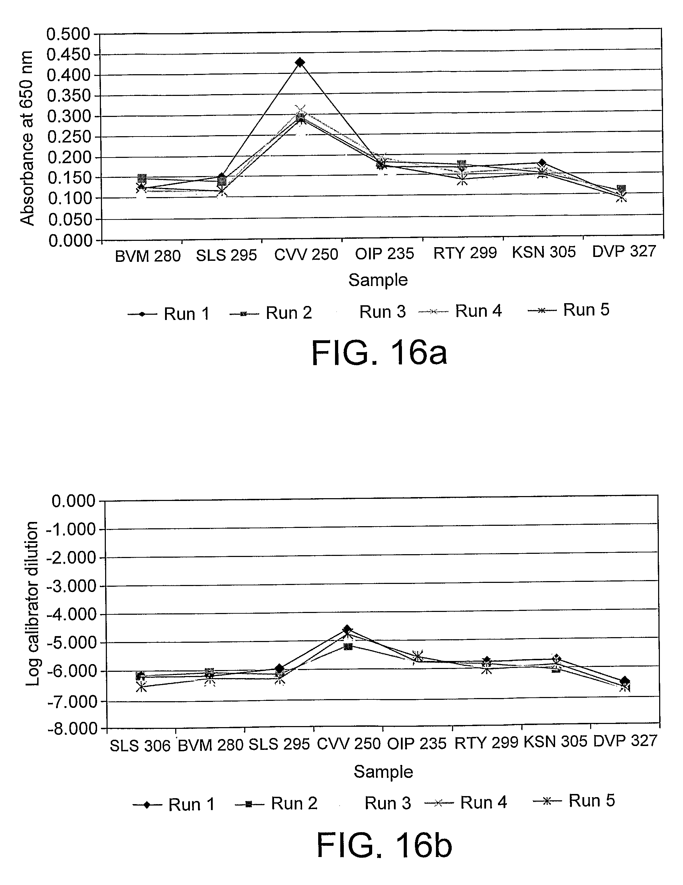

Figure 16: Effect of calibration on the reproducibility of control samples. Autoantibodies against NYESO were measured in 8 control sera on 5 separate occasions. The raw OD values are shown in (a). A calibrator curve was run concurrently and this was used to extrapolate values for control samples (b).

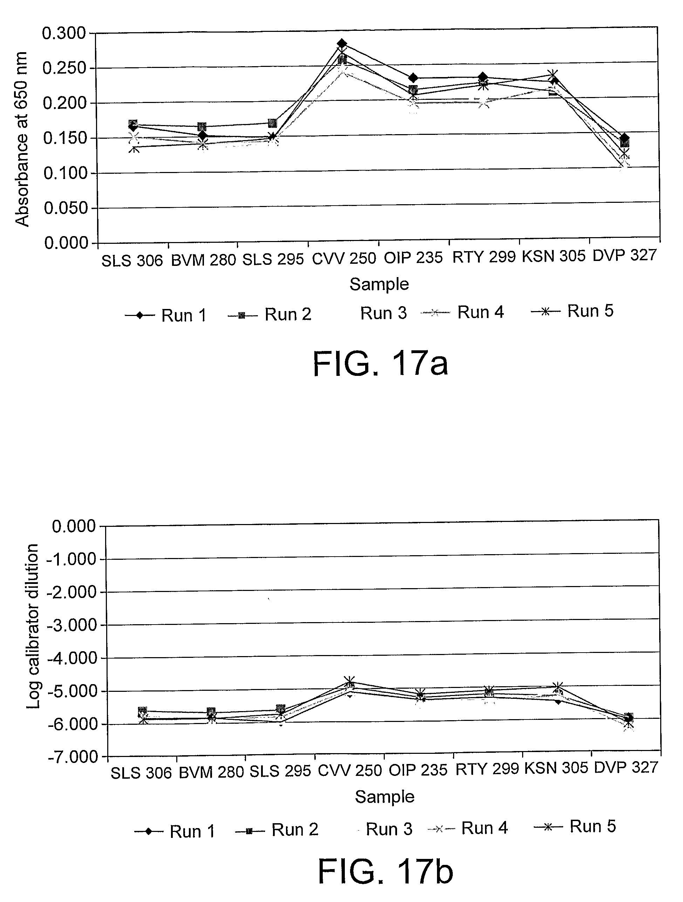

Figure 17: Effect of calibration on the reproducibility of control samples. Autoantibodies against BRCA2 were measured in 8 control sera on 5 separate occasions. The raw OD values are shown in (a). A calibrator curve was run concurrently and this was used to extrapolate values for control samples (b).

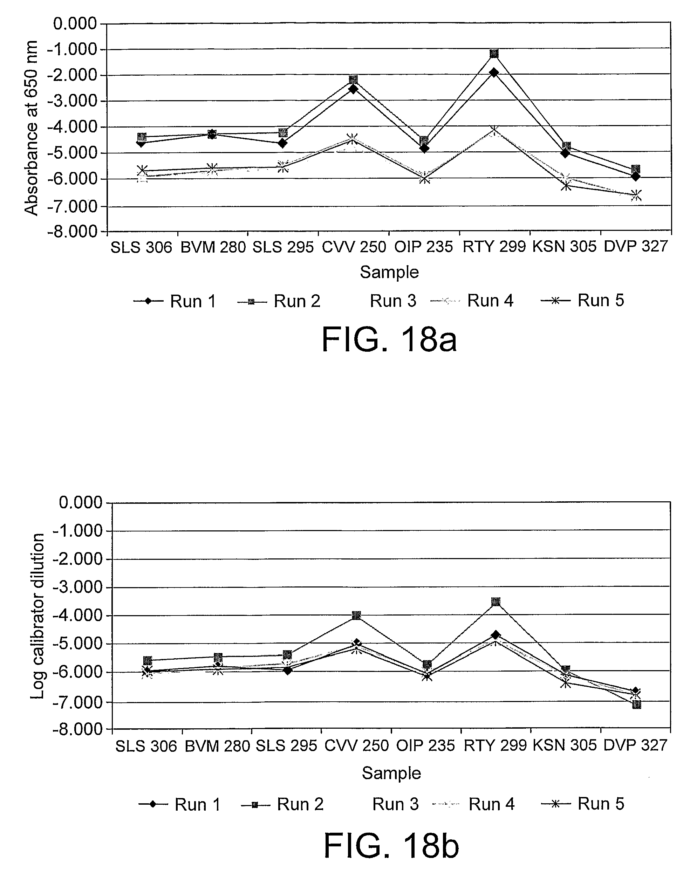

Figure 18: Effect of calibration on the reproducibility of control samples. Autoantibodies against PSA were measured in 8 control sera on 5 separate occasions. The raw OD values are shown in (a). A calibrator curve was run concurrently and this was used to extrapolate values for control samples (b).

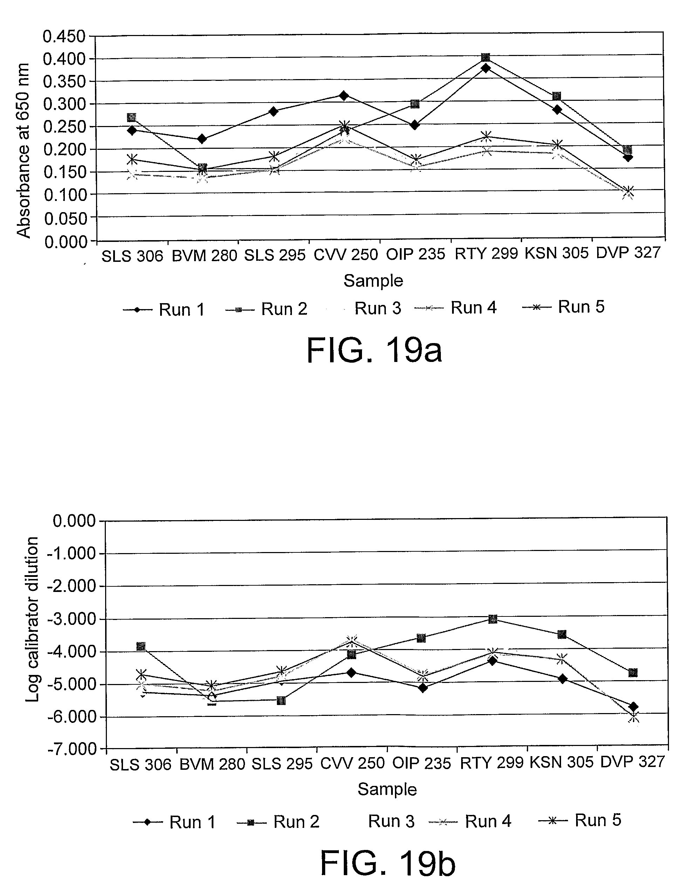

Figure 19: Effect of calibration on the reproducibility of control samples. Autoantibodies against Annexin Xla were measured in 8 control sera on 5 separate occasions. The raw OD values are shown in (a). A calibrator curve was run concurrently and this was used to extrapolate values for control samples (b).

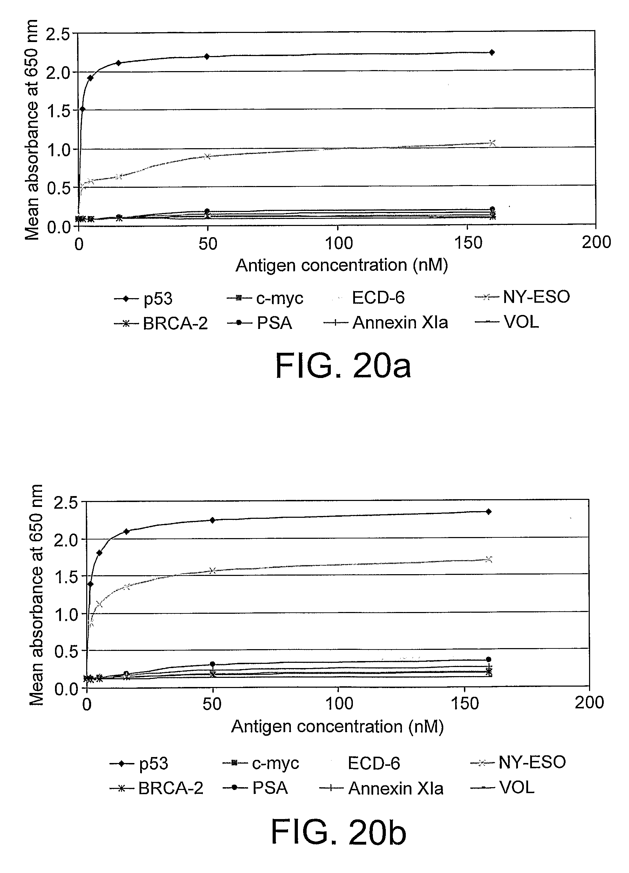

Figure 20: Comparison of serum and drainage fluids as potential calibrator materials for autoantibody assays. Pleural fluid C3 (a) is compared with serum sample 18176 (b) from the same patient.

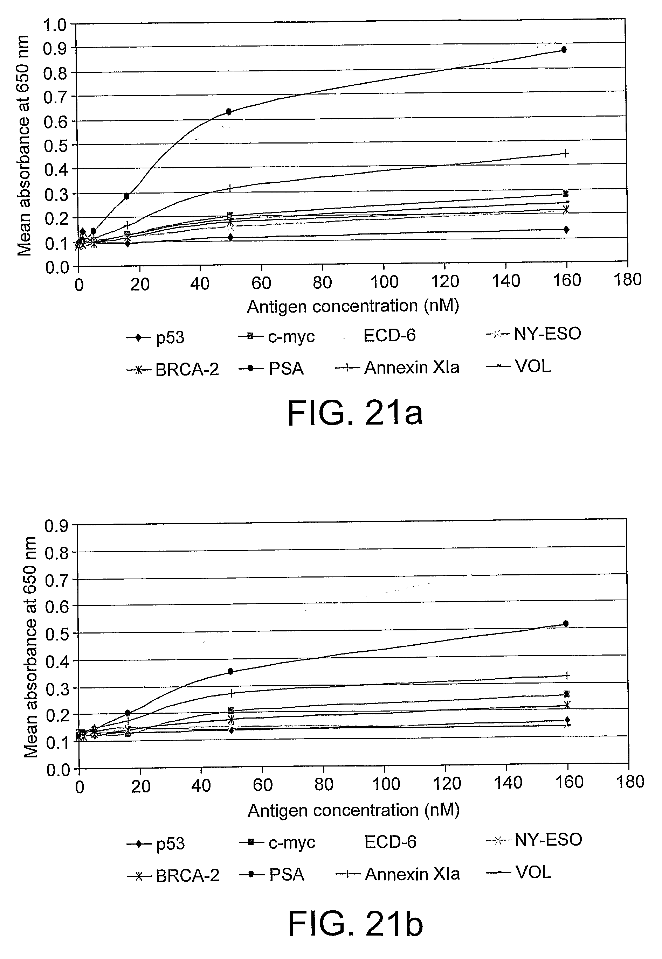

Figure 21: Comparison of serum and drainage fluids as potential calibrator materials for autoantibody assays. Pleural fluid C7 (a) is compared with serum sample 11828 (b) from the same patient.

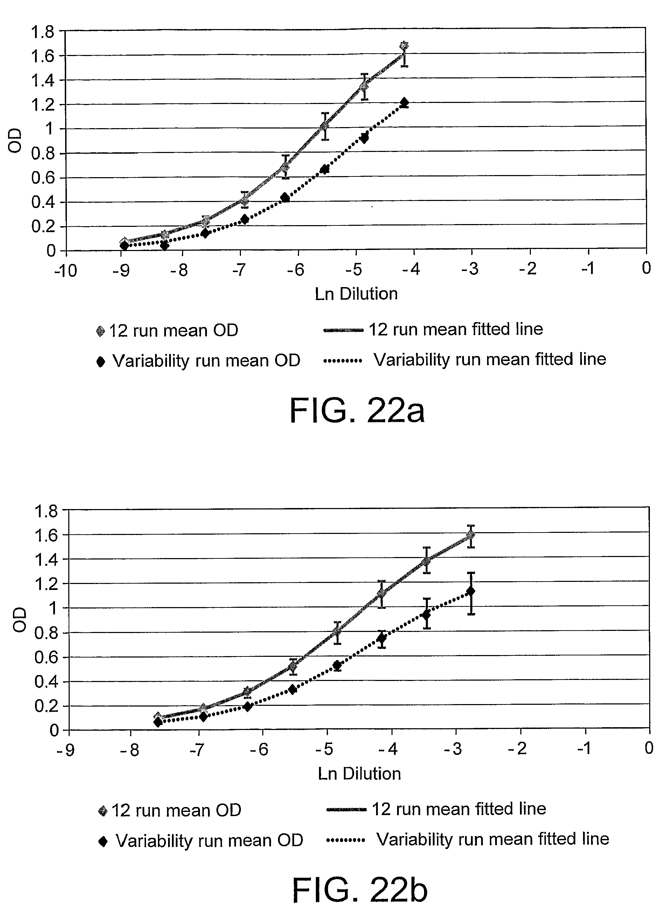

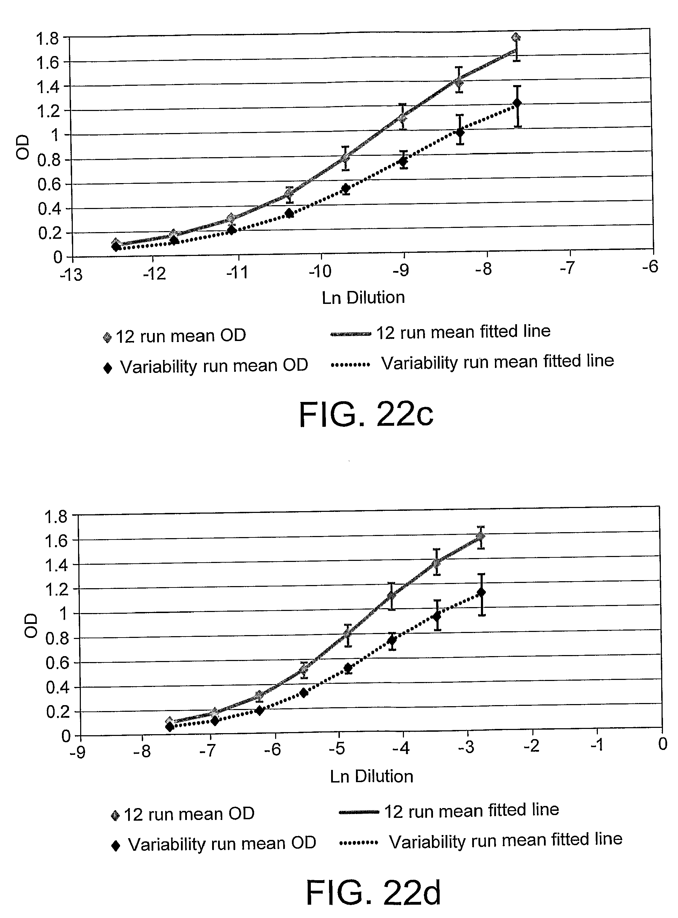

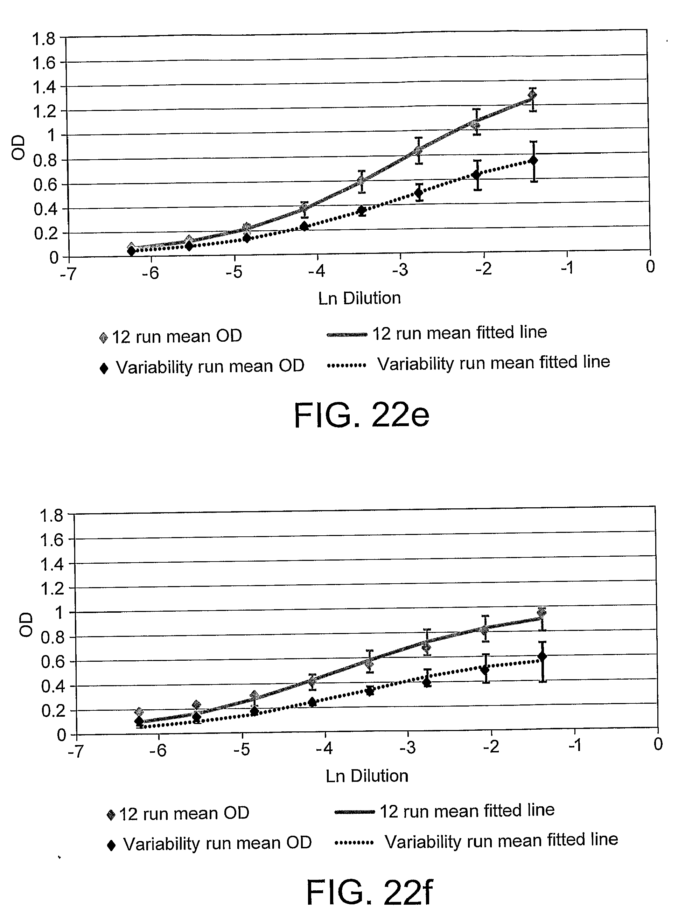

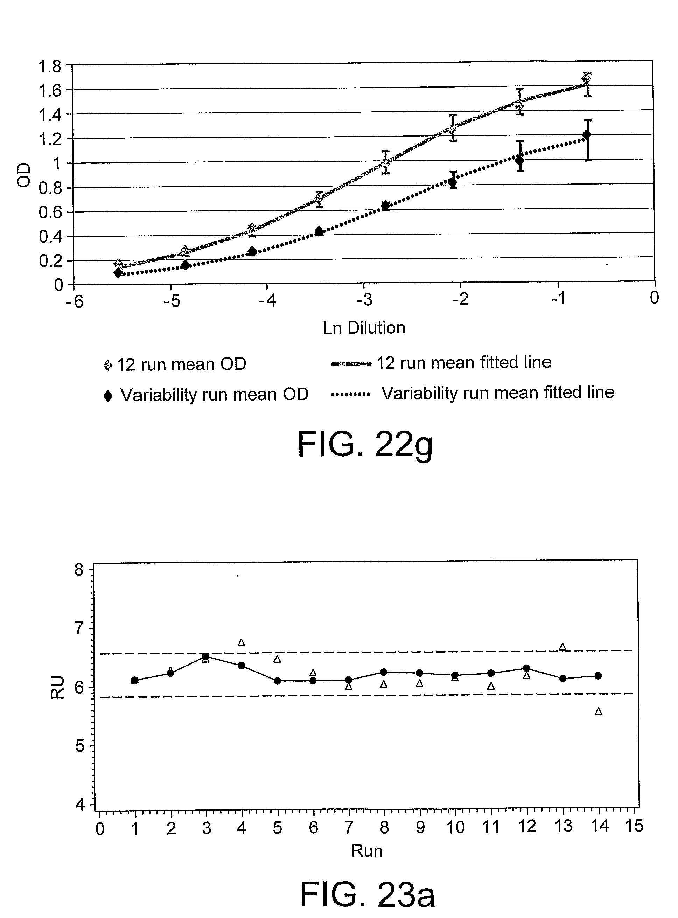

Figure 22: Four-parameter logistic calibrator curves with minimised sum of squared residuals. The 4pl plot is constructed from optical density versus log calibrator dilution. Mean for runs 1 to 12 are shown as solid grey lines and mean for runs 13 and 14 are shown as broken black lines. Error bars represent the standard deviations of the means. Antigen-specific autoantibody assays for p53 (a), c-myc (b), CAGE (c), NY-ESO-1 (d), GBU4-5 (e), Annexin 1 (f) and Annexin 2(g).

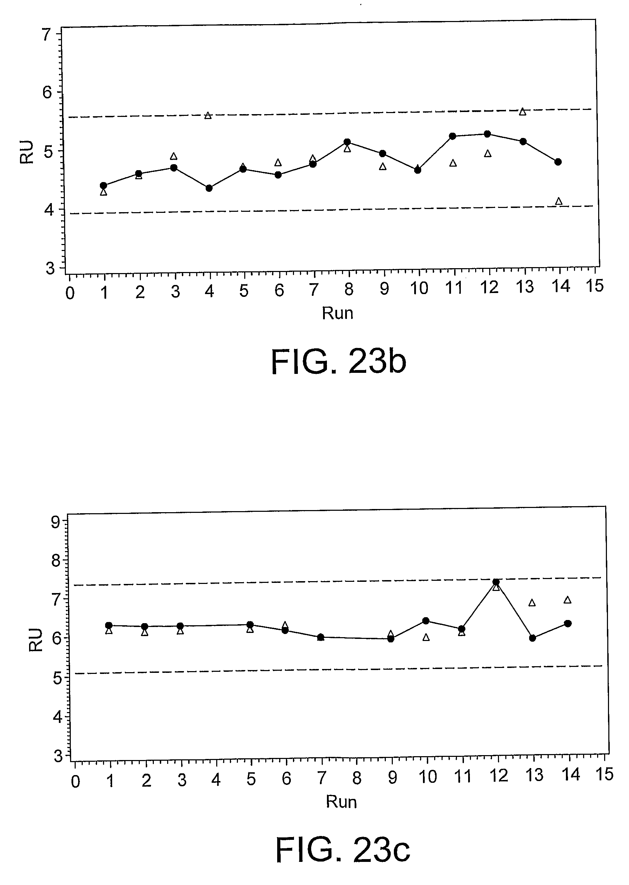

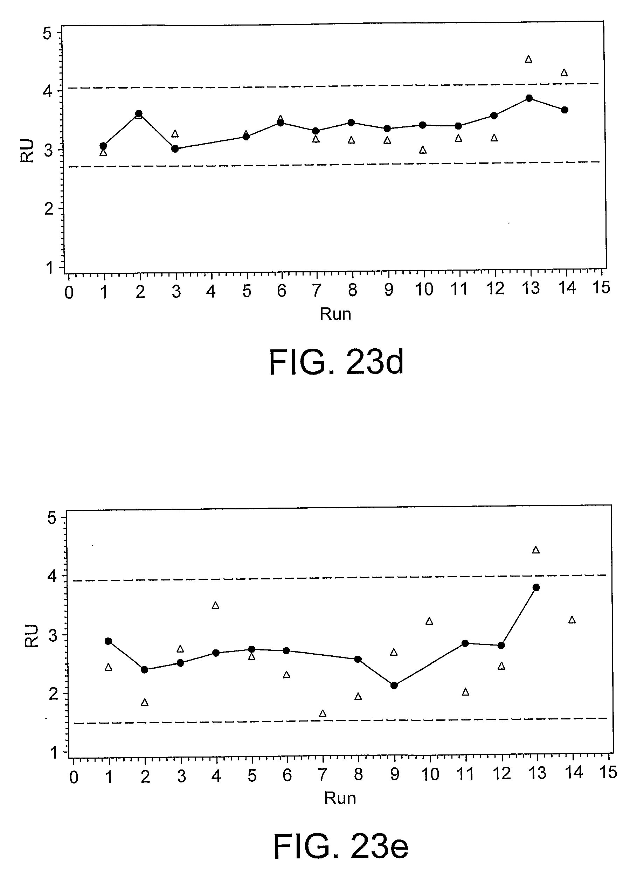

Figure 23: The effect of calibration on the variability of autoantibody measurements made in different assay runs. The results for serum samples were corrected using the antigen-specific calibrator curve for that run. Open triangles = uncalibrated measurements, solid dots = measurements adjusted by calibration, broken lines = mean of the calibrated values plus or minus 3 standard deviations.

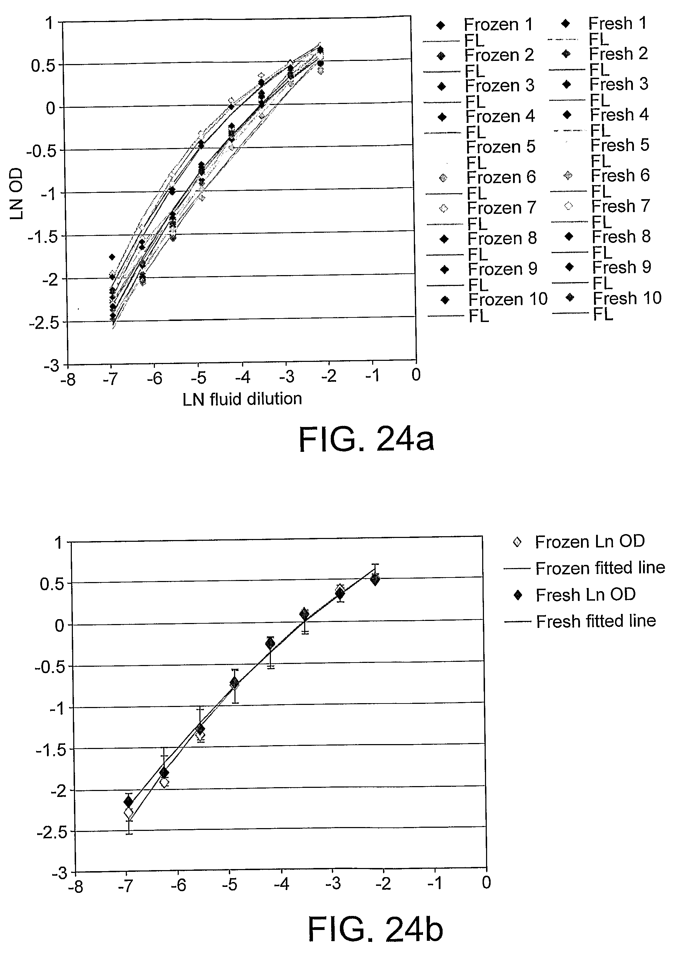

Figure 24: Comparison of frozen aliquots of calibrator series with freshly diluted calibrator series. Calibrator pleural fluid C3 was allowed to react with NYESO antigen. Each pair of fresh and frozen series was run 10 times (a). The average log/log plot is given in (b) with error bars representing standard deviations.

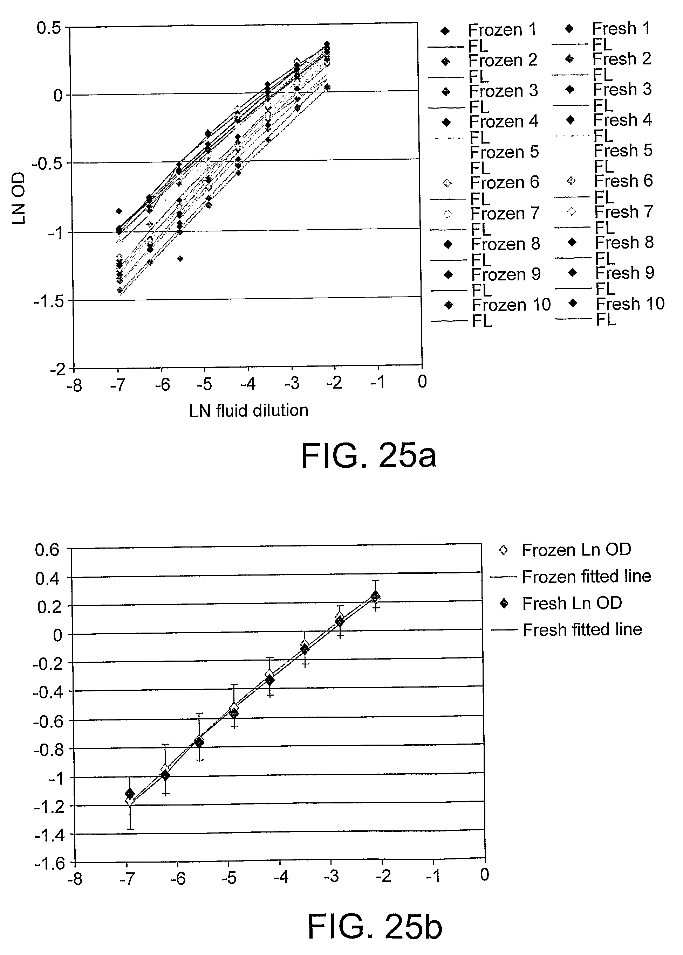

Figure 25: Comparison of frozen aliquots of calibrator series with freshly diluted calibrator series. Calibrator pleural fluid C7 was allowed to react with c-myc antigen. Each pair of fresh and frozen series was run 10 times (a). The average log/log plot is given in (b) with error bars representing standard deviations.

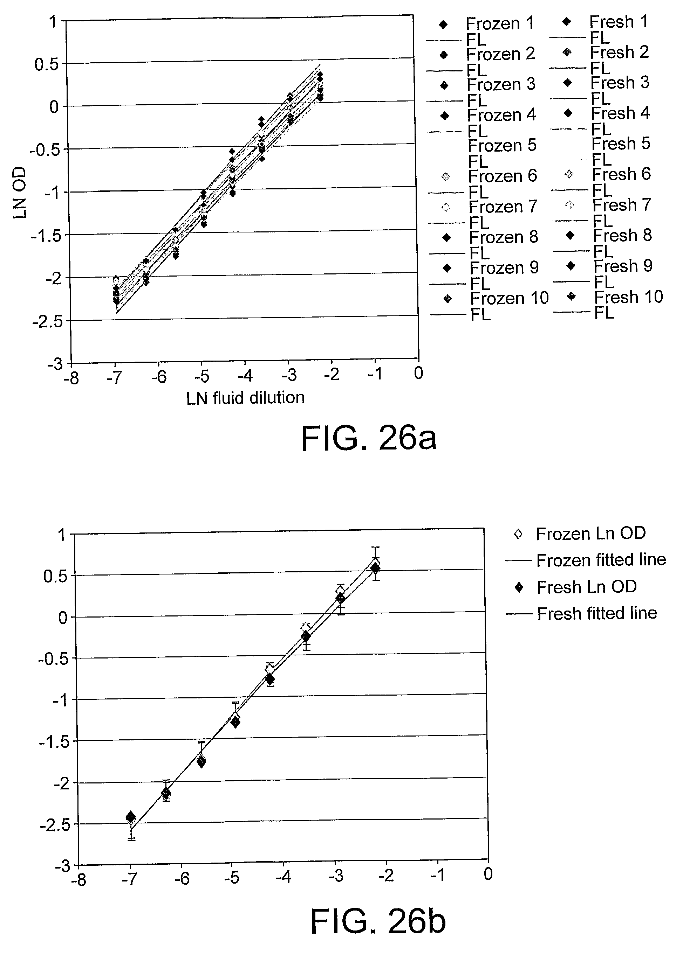

Figure 26: Comparison of frozen aliquots of calibrator series with freshly diluted calibrator series. Calibrator pleural fluid B3258 was allowed to react with p53 antigen. Each pair of fresh and frozen series was run 10 times (a). The average log/log plot is given in (b) with error bars representing standard deviations.

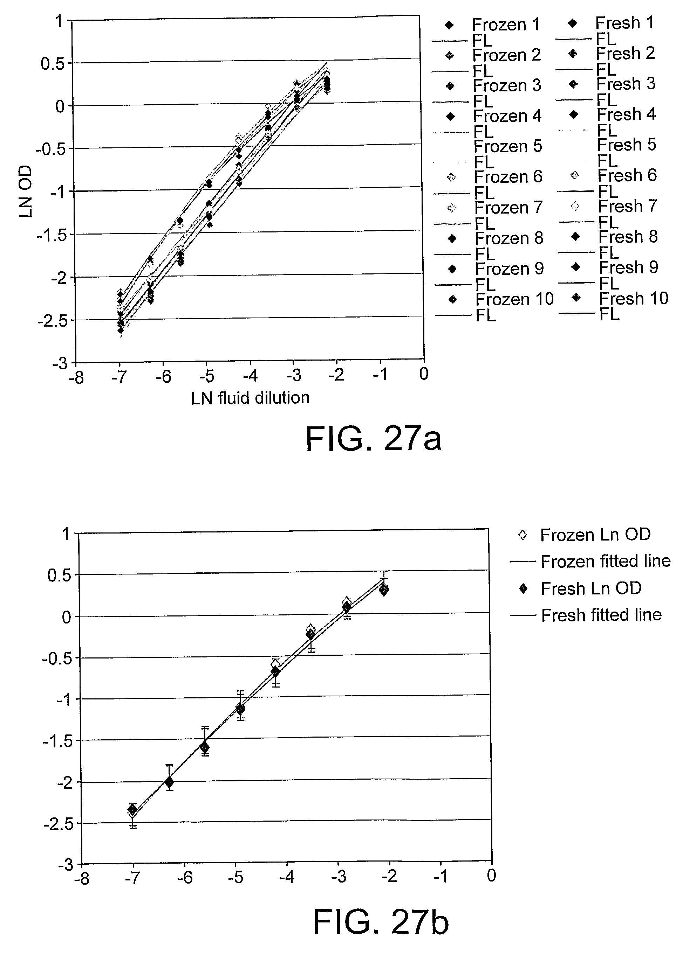

Figure 27: Comparison of frozen aliquots of calibrator series with freshly diluted calibrator series. Calibrator pleural fluid B3258 was allowed to react with PSA antigen. Each pair of fresh and frozen series was run 10 times (a). The average log/log plot is given in (b) with error bars representing standard deviations.

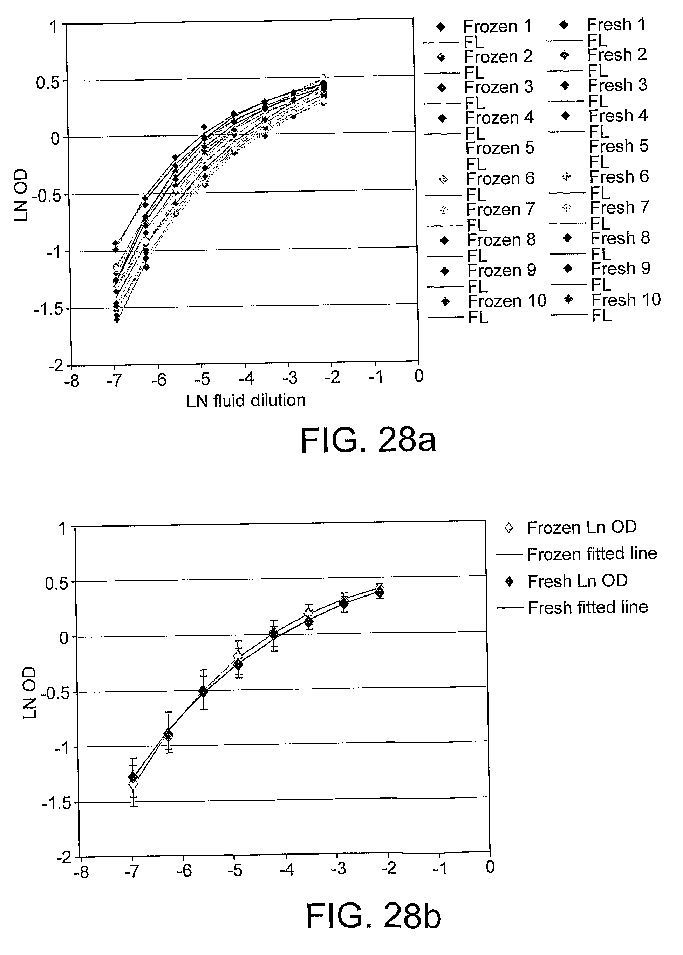

Figure 28: Comparison of frozen aliquots of calibrator series with freshly diluted calibrator series. Calibrator pleural fluid B3258 was allowed to react with Annexin antigen. Each pair of fresh and frozen series was run 10 times (a). The average log/log plot is given in (b) with error bars representing standard deviations.

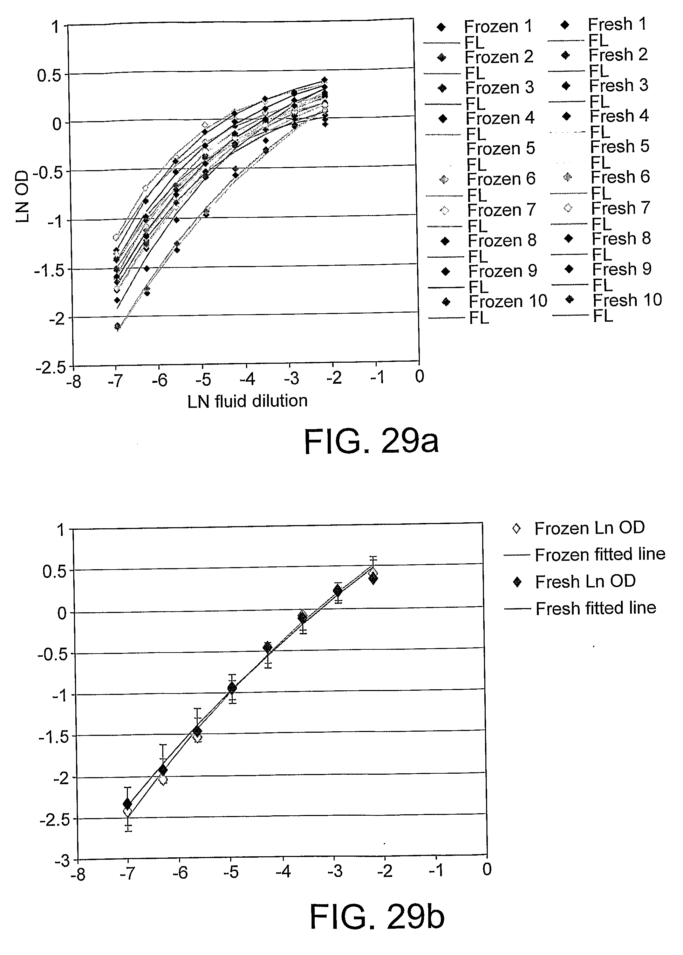

Figure 29: Comparison of frozen aliquots of calibrator series with freshly diluted calibrator series. Calibrator pleural fluid B3255 was allowed to react with BRCA2 antigen. Each pair of fresh and frozen series was run 10 times (a). The average log/log plot is given in (b) with error bars representing standard deviations.

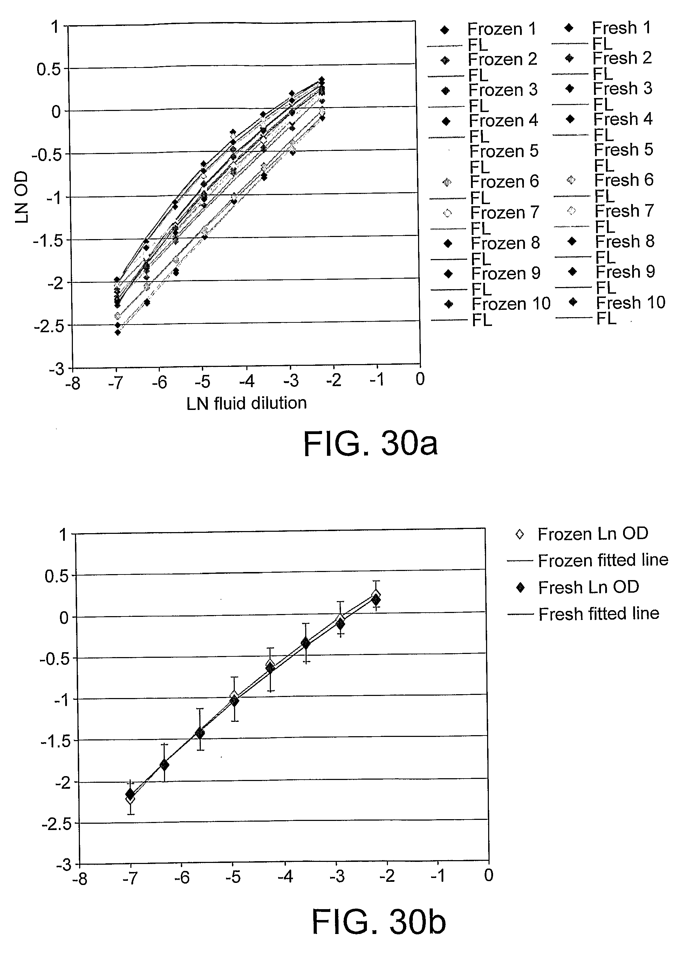

Figure 30: Comparison of frozen aliquots of calibrator series with freshly diluted calibrator series. Calibrator pleural fluid C3 was allowed to react with ECD6 antigen. Each pair of fresh and frozen series was run 10 times (a). The average log/log plot is given in (b) with error bars representing standard deviations.

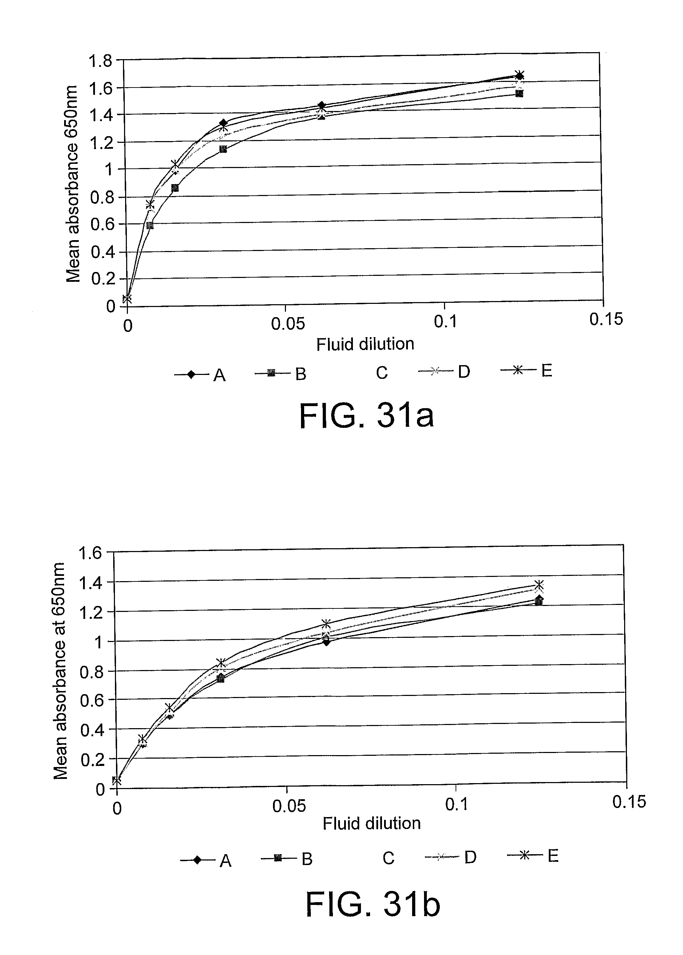

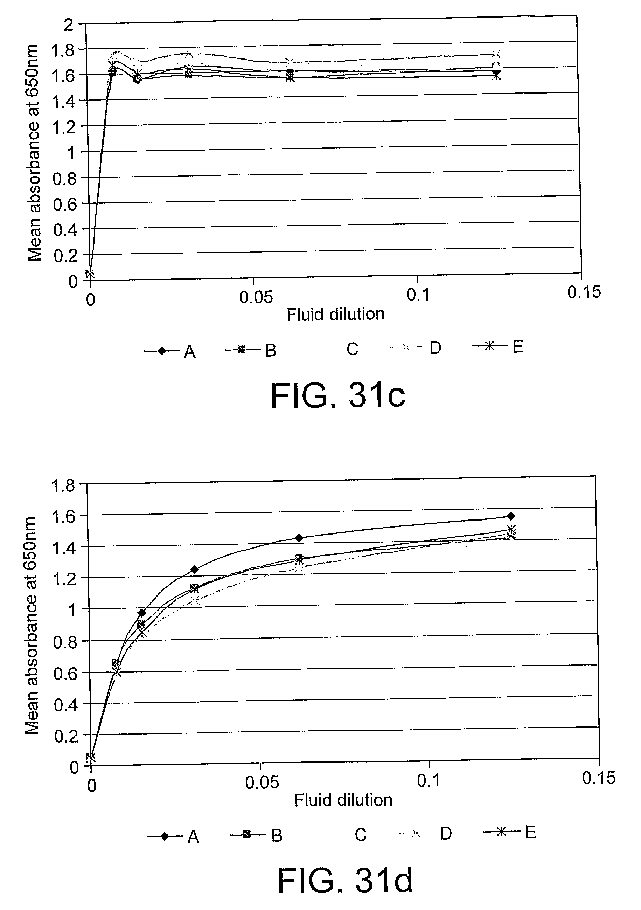

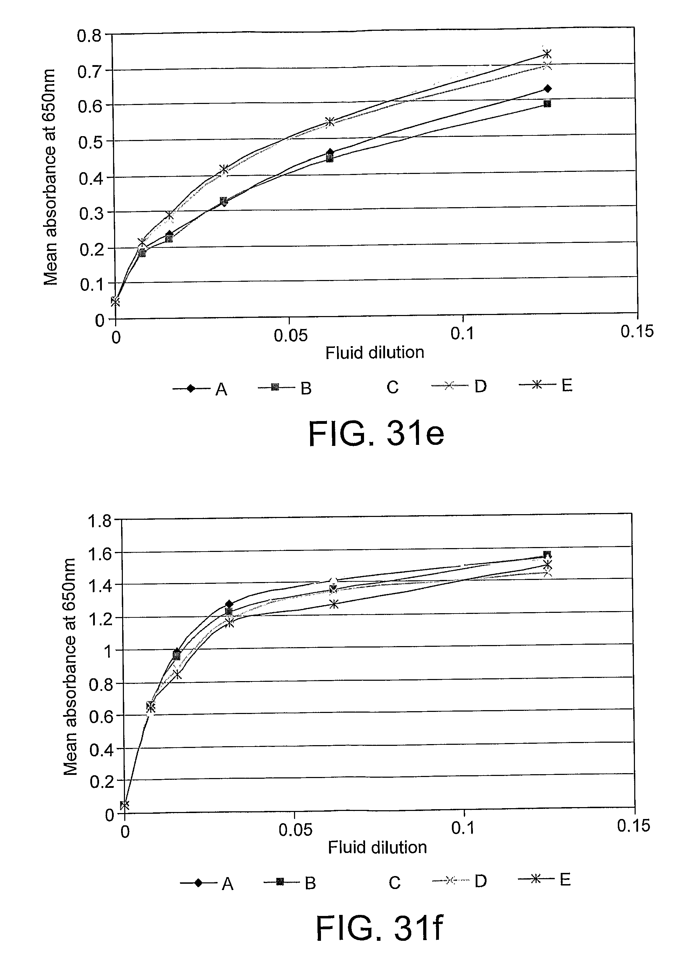

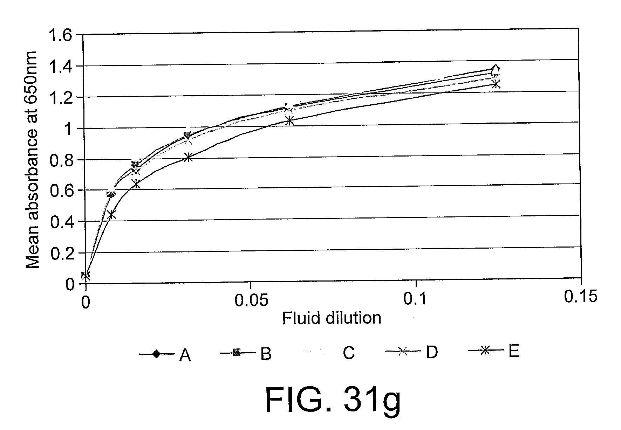

Figure 31: Reactivity of autoantibodies in fluids from patients with different types of cancer with tumour associated antigens.

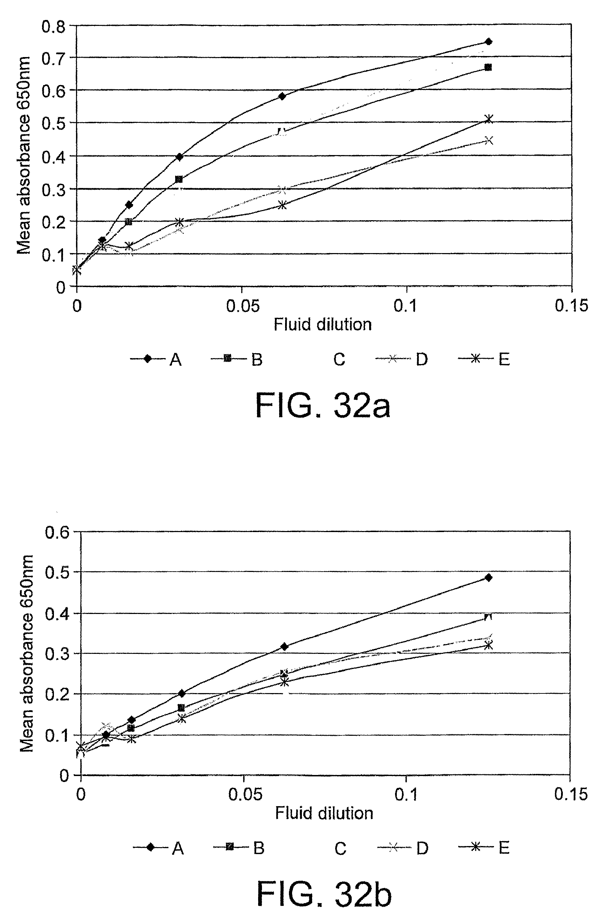

Figure 32: Reaction of a series of dilutions of a pleural fluid from a pancreatic cancer patient with the negative control protein, VOL at 160nM (a) and 50nM (b). The experiment was repeated 5 times on 5 separate days.

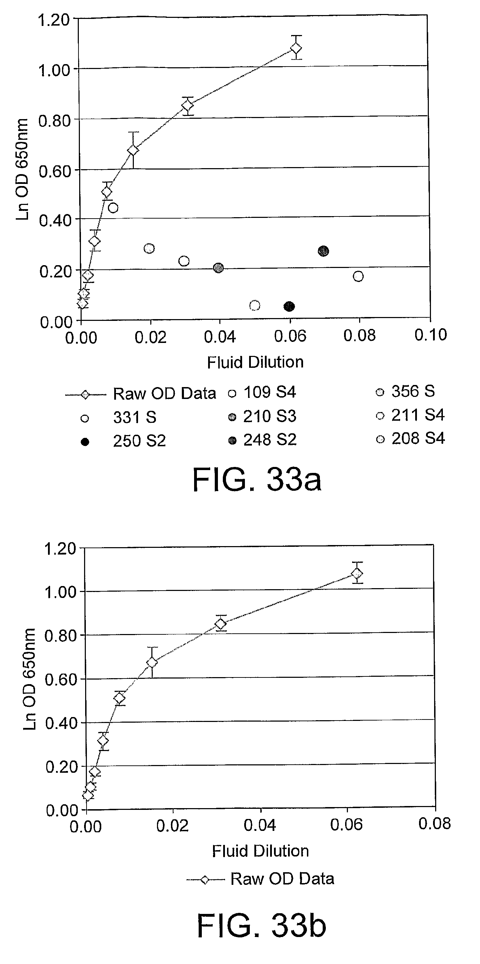

Figure 33: The results of 4 runs of ascites fluid B2993 against 160nM C-myc with standard deviation error bars in both Figures a and b (figure a depicts the OD value of the control serum used in this experiment).

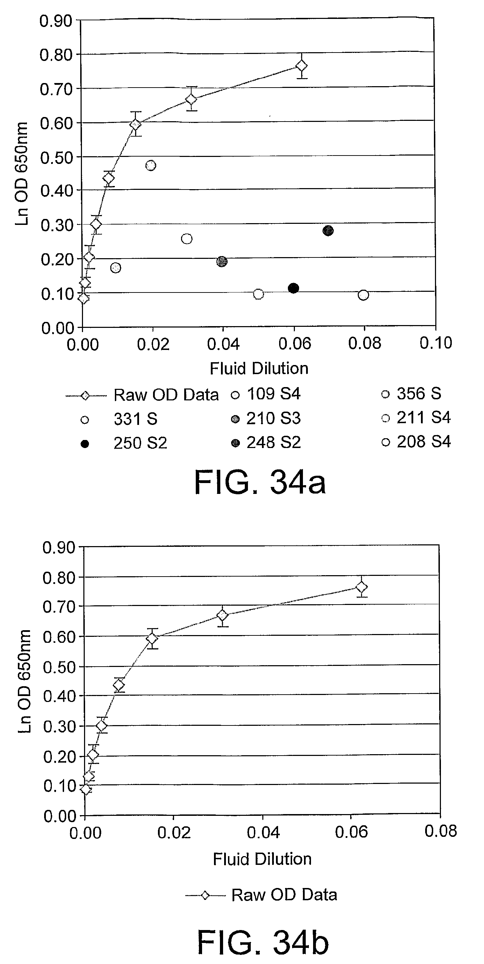

Figure 34: The results of 4 runs of ascites fluid B3259 against 160nM ECD6 with standard deviation error bars in both Figures a and b (figure a depicts the OD value of the control serum used in this experiment).

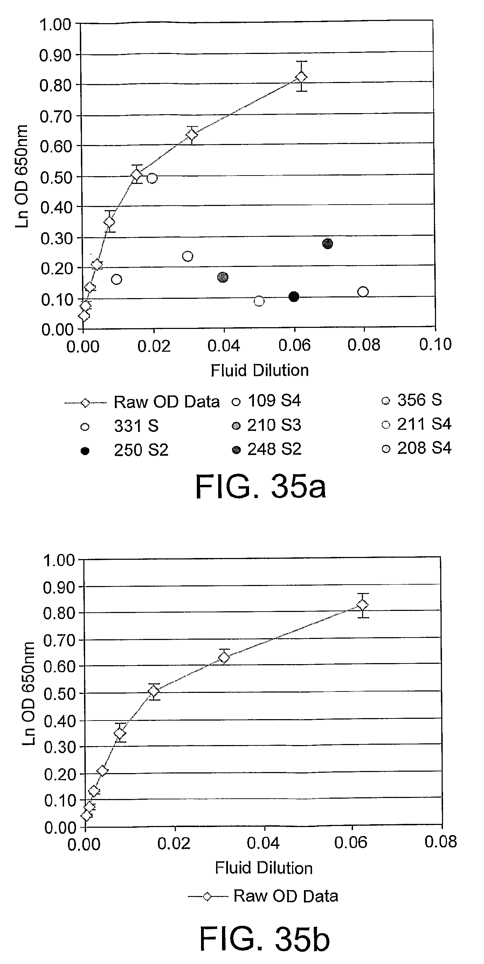

Figure 35: The results of 4 runs of ascites fluid B2993 against 160nM ECD6 with standard deviation error bars in both Figures a and b (figure a depicts the OD value of the control serum used in this experiment).

### Detailed description of the invention

The present invention relates to use of a calibration material comprising a mammalian, and in particular a human, bodily fluid to calibrate an immunoassay for detection of autoantibodies, and in particular human autoantibodies, wherein the calibrator material comprises a drainage fluid, exudate or transudate.

In one embodiment, the calibration material used herein may comprise human bodily fluid as a source of "native human autoantibodies", meaning autoantibodies which have been produced in a human host as a result of natural immunological processes. For the avoidance of doubt, this calibration material does *not* comprise non-human antibodies or any human or humanised antibodies produced exogenously by laboratory techniques, e.g. monoclonal antibodies derived from cultured immune cells.

The calibration material may comprise any human or other mammalian bodily fluid which is a drainage fluid, exudate or transudate which is known to contain autoantibodies of the appropriate immunological specificity, i.e. the calibrator must comprise a mammalian (e.g. a human) bodily fluid which is a known positive for the autoantibody to be detected in the immunoassay. In this regard, it should be known in advance that the calibrator fluid contains autoantibodies which exhibit comparable immunological specificity to the autoantibodies which it is desired to detect using the immunoassay. An advantage of the calibration material of the invention is that it contains native human autoantibodies of substantially equivalent immunological specificity to native autoantibodies found in human serum, in terms of binding to the antigen used as a reagent in the immunoassay it is intended to calibrate.

As illustrated in the accompanying examples, one can determine in advance whether a given sample of human bodily fluid contains autoantibodies of the appropriate immunological specificity by carrying out a test assay using a test antigen. In one embodiment this test assay can use the same antigen and detection methodology as it is intended to use in the immunoassay proper. Bodily fluids which are shown to contain autoantibodies immunologically specific for the test antigen in such a test assay are suitable for use as calibrator materials in subsequent immunoassays using the same test antigen to detect corresponding autoantibodies in patient test samples of unknown autoantibody status. In this context, "test samples" can be defined as samples removed from subjects to be tested for the presence of autoantibodies, wherein the autoantibody status of the patient is unknown prior to testing of the sample.

It is not generally necessary to determine an accurate titer of the amount of autoantibody present in the calibration material prior to use, particularly when using the calibration method of the invention, which utilises multiple dilutions of the calibration material to provide a set of calibration standards. The absolute amount of autoantibody present in the set of calibration standards need not be accurately determined, provided that the set of calibration standards covers the normal range of autoantibody titers which one would expect to encounter when testing patient test samples of unknown antibody status in the immunoassay proper. This can be determined empirically by testing a range of diluted calibration samples in parallel in comparison to typical patient test samples.

The calibration material may simply consist of the human bodily fluid in the form in which it is isolated from the human body (e.g. "neat" pleural or ascites fluid) or the bodily fluid may be admixed or diluted with other components to form a calibration material prior to use. Typically a dilution series of the fluid in a suitable buffer is prepared to provide a set of calibration standards. Suitable dilution buffers for preparation of the calibration standards include, for example, a high salt buffer of PBS + 0.5M NaCl + 0.1% casein + 0.1% Tween 20 (referred to in the examples as HSBT) or PBS containing 1% BSA. The invention therefore contemplates use of calibrator materials which consist of a mammalian (e.g. human) bodily fluid of a type described herein admixed with one of these dilution buffers. Particularly useful calibrators consist of human pleural fluid or human ascites fluid, which may be obtained from one or more human cancer patients, admixed with HSBT. Alternatively, normal serum could be used as a calibrator diluent. It is also contemplated to concentrate the bodily fluid or (semi) purify the antibodies and then dilute this material to provide calibration standards. Additional components may be added to the calibration material, for example to improve stability during long term storage.

Samples of calibration material diluted in appropriate dilution buffer may be dispensed in aliquots and stored prior to use. Conveniently, ready-diluted aliquots of calibration material may be stored frozen at -20ºC or -80°C and thawed prior to use. The inventors have shown that pleural and ascites fluids are stable to storage at -20ºC and can be stored frozen for extended periods without loss of autoantibody reactivity. Pre-dilution and aliquotting of calibration standards prior to long term frozen storage is convenient and avoids reproducibility errors and numerous freeze-thaw cycles.

The use of known positive samples as calibrators for immunoassays is fairly routine practice in the field of immunoassays for detection of antigen. However, it is difficult to provide suitable calibration materials which are known positive samples containing antibodies of appropriate specificity when the target of the assay is an antibody, rather than an antigen. The invention addresses this problem by use of calibration material comprising bodily fluid which is known in advance to contain antibodies of the appropriate specificity.

Generally it is preferred not to use bodily fluids which are or comprise "blood products", such as whole blood, plasma or serum, as the basis of the calibrator material. Instead, the calibrator material comprises bodily fluids which are drainage fluids, exudates or transudates, and includes such materials produced during or as a result of disease. In non-limiting embodiments, the bodily fluid may be selected from: pleural effusion, ascites, hydrocoele, wound drainage fluid, inflammatory or non-inflammatory synovial fluid, seroma, nipple aspirate fluid, pericardial effusion, bile, pancreatic secretions, etc. The fluid may be obtained from a human subject or from a non-human mammalian subject, including for example dogs and non-human primates.

In certain embodiments, the calibration material may comprise bodily fluid isolated from a body cavity or space in which a tumour is or was present or with which a tumour is or was associated. In this regard, the term "body cavity or space" includes any body cavity or space, whether it be a natural cavity or a space or cavity arising as a result of diseases or medical intervention including collapsed or former cavities. The fluid is derived from such a cavity or space in which a tumour is or was present or with which a tumour is or was associated. Preferably the "bodily fluid derived from a body cavity" will be a tumour-induced body fluid, meaning a body fluid which is produced during the disease process, for example in response to or as a consequence of the presence of tumour cells. In this connection, exemplary "body cavity" fluids are ascites, pleural effusion, seroma, hydrocoele and wound drainage fluid.

For the avoidance of doubt "bodily fluids derived from a body cavity or space" do not include blood products derived from the systemic circulation, such as whole blood, serum or plasma.

Pleural fluid and ascites fluid are particularly useful sources of calibration material for use according to the invention since they are typically obtained in large volume and removed from patients as part of the therapeutic strategy. This fluid, which would otherwise be discarded, is a valuable source of calibration material. As outlined above, the inventors have demonstrated that human "body cavity" fluids such as pleural fluid and ascites are suitable calibration materials for immunoassays for detection of autoantibodies in human serum, since these fluids contain autoantibodies which are comparable to those present in human serum, both in terms of immunological specificity of binding to antigen and also antibody isotype. The latter is important since it enables the same detection system to be used for autoantibodies in the calibration material and autoantibodies of equivalent antigen binding specificity present in patient serum test samples.

The calibration material may comprise bodily fluid, and more specifically "body cavity" fluid such as pleural fluid or ascites fluid, collected from one or more cancer patients. In this context the term "cancer patient" includes an individual previously diagnosed as having cancer, including but not limited to colon cancer, ovarian cancer, lung cancer, liver cancer, pancreatic cancer, oesophageal cancer, gastric cancer, renal cancer, bladder cancer, endometrial cancer, lymphoma and leukaemia or breast cancer. The fluid may be taken from a single patient or fluids obtained from two or more patients may be pooled together. Fluid samples may be pooled from two or more patients having the same or different stages of the same or different types of cancers. It is also contemplated to pool different types of bodily fluids from a single or multiple cancer patients.

A calibration material prepared from bodily fluid taken from cancer patient(s) with a particular type of cancer may be used to assist in the diagnosis of the same types of cancers or different types of cancers in other individuals. As illustrated in the accompanying examples, native human autoantibodies specific for tumour marker proteins are present in pleural fluids taken from patients with colon cancer, ovarian cancer, lung cancer, liver cancer, pancreatic cancer and breast cancer. Once the presence of autoantibodies of the required immunological specificity has been established using a test assay, such fluids can be used to calibrate immunoassays to test for autoantibodies of equivalent immunological specificity in test samples from patients with other types of cancer, e.g. immunoassays for autoantibodies in breast cancer serum can be calibrated using calibration material comprising pleural fluid (and other body cavity fluids) from patient(s) with colon cancer, ovarian cancer, lung cancer, liver cancer, or pancreatic cancer.

In one embodiment a stock of calibration material prepared from a patient diagnosed with cancer may be used to calibrate an immunoassay carried out at a later date to assess the immune status of the same patient or a different patient, for example to monitor disease progression and/or to assess the effectiveness of a course of anti-cancer treatment in that patient.

In use, the calibration material of the invention can be used to calibrate immunoassays for detection of autoantibodies carried out according to known methods. Typically, the immunoassay may take the form of a direct, sandwich or competitive ELISA, but other assay methodologies are also within the scope of the invention. General features of immunoassays for detection of human anti-tumour marker autoantibodies are described in WO 99/58978 and WO 2006/126008. The calibrator material provided by this invention can be used to calibrate the assays described in WO 99/58978 and WO 2006/126008.

The calibration material described herein, and immunoassay kits comprising sets of calibration standards comprising this calibration material, can be used to calibrate an immunoassay for any type of autoantibody which serves as a marker of disease state or disease susceptibility, wherein the disease in question has the potential to produce/induce formation of a bodily fluid of the type described herein, comprising autoantibodies of comparable immunological specificity to the autoantibodies which serve as the disease marker.

Examples of diseases which are typically associated with the production of bodily fluids containing autoantibodies include cancers of the types listed herein. As explained above, bodily fluids obtained from cancer patients, and in particular "body cavity fluids" such as pleural fluid, ascites fluid, hydrocoele, seroma, wound drainage fluid etc., provide a useful source of positive calibration material containing autoantibodies specific for tumour-markers. This calibration material can therefore be used to calibrate immunoassays for detection of cancer or early neoplastic disease in patient test samples (e.g. patient *serum* samples of unknown autoantibody status). Such assays (for detection of anti-tumour marker autoantibodies in patient test samples) can be carried out using the methods described in WO 99/58978 and WO 2006/126008, or modifications thereof.

It should be understood, however, that the invention is not limited to the use of cancer-derived fluids, nor indeed to the detection of autoantibodies to tumour-markers, although this is an important embodiment. Another group of diseases associated with the production of bodily fluids (other than serum, whole blood or plasma) containing autoantibodies characteristic of the disease are the benign autoimmune diseases. The invention therefore contemplates use of bodily fluids obtained from mammalian (e.g. human) subjects with benign autoimmune disease as calibration materials for immunoassays for detection of autoantibodies which are markers of the autoimmune disease. Examples of such autoimmune diseases include rheumatoid arthritis (RA), systemic lupus erythematous (SLE), primary biliary cirrhosis (PBC), autoimmune thyroiditis (e.g. Hashimoto's thyroiditis), autoimmune gastritis (e.g. pernicious anaemia), autoimmune adrenalitis (e.g. Addison's disease), autoimmune hypoparathyroidism, autoimmune diabetes (e.g. Type 1 diabetes) or myasthenia gravis.

In the case of rheumatoid arthritis, the calibrator material may comprise or consist of an exudate associated with the disease process, typically a fluid accumulating in a joint, such as inflammatory synovial fluid isolated from the knee of a patient with RA.

In the case of systemic lupus erythematous (SLE), the calibrator material may comprise or consist of ascites fluid obtained from patients with SLE (see Lacconi et al. Internet Journal of Radiology, ISSN: 1528-8404). In this regard, it should be noted that not all ascites fluids (or indeed other body cavity fluids such as pleural effusions) are associated with the presence of a tumour.

In the case of biliary cirrhosis, the calibrator material may comprise or consist of ascites fluid obtained from biliary cirrhosis patients.

The general features of immunoassays, for example ELISA, radioimmunoassays and the like, are well known to those skilled in the art (see Immunoassay, E. Diamandis and T. Christopoulus, Academic Press, Inc., San Diego, CA, 1996). Immunoassays for the detection of antibodies having a particular immunological specificity (e.g. autoantibodies having immunological reactivity with a given antigen, such as a tumour marker protein) generally require the use of a reagent comprising an antigen which exhibits specific immunological reactivity with the antibody under test. Depending on the format of the assay, this reagent may be immobilised on a solid support. A test sample to be tested for the presence of the antibody is brought into contact with the reagent and if antibodies of the required immunological reactivity are present in the test sample they will immunologically react with the reagent to form autoantibody-reagent complexes which may then be detected or quantitatively measured. Such immunoassays are typically calibrated by carrying out parallel assays using the same reagents used to detect (auto)antibodies in the test sample, but replacing the test sample with one or more calibration standards, which are samples of calibration material known to contain (auto)antibodies of the appropriate immunological specificity.

The preferred calibration method using the calibration material of the invention utilises a set of calibration standards, typically serial dilutions of the calibration material of the invention, which are tested against one or more known concentrations of antigen. In a typical "sandwich" ELISA the antigen having specificity for the autoantibodies under test is immobilised on a solid surface (e.g. the wells of a standard microtiter assay plate, or the surface of a microbead) and a sample of calibrator (or test sample to be tested for the presence of autoantibodies) is brought into contact with the immobilised antigen. Autoantibodies of the desired specificity present in the calibrator material will bind to the immobilised antigen. The bound autoantibody/antigen complexes may then be detected using any suitable method.

The invention therefore provides a method of calibrating an immunoassay for detection of autoantibodies which comprises:
(a) contacting each of a plurality of different dilutions of a calibration material comprising a human or other mammalian bodily fluid with an antigen (immunologically) specific for an autoantibody, wherein said human bodily fluid is known to contain autoantibodies immunologically specific for the antigen, wherein the calibrator material comprises a drainage fluid, exudate or transudate;
(b) detecting the amount of (immunologically) specific binding between said antigen and autoantibody present in the calibration material; and
(c) plotting or calculating a curve of the amount of said specific binding versus the dilution of the calibration material for each dilution of calibration material used in step (a), thereby calibrating an immunoassay using said antigen for detection of said autoantibody.

The precise methodology used to detect specific binding in step (b) is not limiting to the invention. In one embodiment a labelled secondary anti-human immunoglobulin antibody, which specifically recognises an epitope common to one or more classes of human immunoglobulins, is used to detect the autoantibody/antigen complexes. Typically the secondary antibody will be anti-IgG or anti-IgM. The secondary antibody is usually labelled with a detectable marker, typically an enzyme marker such as, for example, peroxidase or alkaline phosphatase, allowing quantitative detection by the addition of a substrate for the enzyme which generates a detectable product, for example a coloured, chemiluminescent or fluorescent product. Other types of detectable labels known in the art may be used with equivalent effect.

The concentration of antigen used in step (a) is selected to give a broad dynamic range in terms of the binding measurements obtained in step (b), in order to provide calibration for a wide range of autoantibody measurements. This is a particularly important consideration in relation to immunoassays for detection of anti-tumour marker autoantibodies, which by definition are polyclonal and exhibit patient-to-patient variation in terms of the strength of antigen/autoantibody binding as well as the absolute amount of autoantibody present. The concentration of antigen used will typically be greater than 20nM, and more particularly be in the range of from 20nM to 180nM, or in the range of from 50nM to 160nM.

As many dilutions of the calibration material may be tested as are needed to construct a broad calibration curve in part (c). Typically at least 6 separate dilutions of the calibration material will be tested at each antigen concentration used, but this number is not intended to be limiting.

A preferred use of the calibration material described herein is as a calibrator for immunoassays for detection of native human autoantibodies immunologically specific for human tumour markers, these autoantibodies typically being cancer-associated.

The development and progression of cancer in a patient is generally found to be associated with the presence of markers in the bodily fluid of the patient, these "tumour markers" reflecting different aspects of the biology of the cancer (see Fateh-Maghadam, A. & Steilber, P. (1993) Sensible use of tumour markers. Published by Verlag GMBH, ISBN 3-926725-07-9; Harris et al., J Clin Oncol., 25: 5287-5312, 2007; Voorzanger-Rousselot and Garnero, Cancer Treatment Reviews, 31: 230-283, 2007). Tumour markers are often found to be altered forms of wild-type proteins expressed by "normal" cells, in which case the alteration may be a change in primary amino acid sequence, a change in secondary, tertiary or quaternary structure or a change in post-translational modification, for example, abnormal glycosylation. In addition, wild-type proteins which are up-regulated or over-expressed in tumour cells, possibly as a result of gene amplification or abnormal transcriptional regulation, may also be tumour markers.

Differences between a wild type protein expressed by "normal" cells and a corresponding tumour marker protein may, in some instances, lead to the tumour marker protein being recognised by an individual's immune system as "non-self" and thus eliciting an immune response in that individual. This may be a humoral (i.e B cell-mediated) immune response leading to the production of autoantibodies immunologically specific to the tumour marker protein. Autoantibodies are naturally occurring antibodies directed to an antigen which an individual's immune system recognises as foreign even though that antigen actually originated in the individual. They may be present in the circulation as circulating free autoantibodies or in the form of circulating immune complexes consisting of autoantibodies bound to their target tumour marker protein.

The term "cancer-associated" anti-tumour marker autoantibodies refers to autoantibodies which are characteristic of the cancer disease state, and which are directed against epitopes present on forms of tumour marker proteins which are preferentially expressed in the cancer disease state.

Typically, the tumour marker antigens used to detect anti-tumour marker autoantibodies comprise recombinant tumour marker proteins (expressed in bacterial, insect, yeast or mammalian cells) or chemically synthesised tumour marker antigens, which may comprise substantially whole tumour marker proteins, or fragments thereof, such as short peptide antigens. Other potential sources of tumour-associated proteins for use as the basis of immunoassay reagents for the detection of anti-tumour auto-antibodies include cultured tumour cells (and the spent media used for their growth), tumour tissue, and serum from individuals with neoplasia, or other bodily fluids from one or more cancer patients (as described in WO 2004/044590).

The calibration material described herein may be used to calibrate immunoassays for detection of a wide range of anti-tumour marker autoantibodies against different tumour markers, irrespective of the nature of the antigen used in such assays. A key feature of the calibration material used in this invention (and especially calibration material comprising pleural fluid or ascites fluid from one or more cancer patients) is that it contains autoantibodies which closely resemble those present in cancer patient test samples (e.g, cancer patient serum) in terms of antigen binding specificity. This calibration material may be used with recombinant tumour marker antigens, synthetic peptide tumour marker antigens or purified tumour marker native antigens.

The invention is not intended to be limited with respect to the target of the immunoassay, i.e. the specificity of the target autoantibody which it is intended to detect. The calibration material described herein can be used to calibrate an immunoassay for any autoantibody present in the calibration material itself. A single calibration material may contain a number of different autoantibodies of different immunological specificity and so the same material may be used to calibrate a number of different assays. By way of example, samples of human pleural effusion have been shown in the current examples to contain autoantibodies to a range of tumour markers, including p53, c-myc, ECD-6 (HER2/neu extracellular fragment), NY-ESO1, BRCA2, PSA and Annexin X1-A.

There is described a calibration material which can be used in order to quantitate the amount of tagged protein bound to a solid surface, such as the wells of a microtiter plate, due to the presence in the calibration material of native autoantibodies immunologically specific to a peptide tag component of the "tagged" protein, such as for example a histidine tag or biotin tag. It has been observed that certain samples of pleural fluid isolated from cancer patients contain antibodies immunologically specific for histidine and/or biotin tags attached to recombinant tumour marker antigens. These pleural fluids can therefore be used to provide a generic quantitative ELISA for recombinant proteins bearing histidine and/or biotin tags which utilises a native human antibody specific for the tag, in combination with a labelled anti-human secondary antibody. This method provides certain advantages over the use of murine monoclonal antibodies to quantitate tagged antigen bound to a solid support in the overall context of immunoassays for anti-tumour marker autoantibodies, since it uses the same reporter system as that used to measure native human autoantibodies specific for the tumour marker antigen itself. Thus, within a single assay plate one can run an assay to quantitate the amount of tagged recombinant tumour marker antigen bound to the plate using calibration material containing native autoantibodies to the histidine or biotin tag portion of the antigen and in parallel run a set of calibration standards for binding of the same tagged recombinant antigen to native anti-tumour marker autoantibodies and use the same reporter system for both assays.

There is described a method of quantitating the amount of protein bound to a solid surface, wherein said protein comprises a tag, the method comprising: contacting the solid surface to be tested for the presence of said protein with a reagent material comprising a human bodily fluid, wherein said bodily fluid is known to contain a native human antibody immunologically specific for the tag, and measuring the amount of specific binding between the native human antibody and the tag, thereby quantitating the amount of said protein present on the surface.

In this context, the term "tag" refers to chemical moiety attached to the protein which is not present in any naturally expressed form of the protein. The tag can be a polypeptide, in which case the tag consists of a sequence of amino acids which is non-contiguous with the amino acid sequence of any naturally expressed form of the protein.

The protein to be quantitated on the solid surface is typically a recombinantly expressed protein. Examples of tags commonly attached to recombinantly expressed proteins include biotin tags and histidine tags. As illustrated in the accompanying examples, about 10% of the human population contain native human antibodies which are immunologically specific for biotin. Human individuals with native antibodies specific for histidine tags can also be identified within the normal human population.

It will be understood that statistical and mathematical analyses of calibration curve data obtained according to the present invention can include, but need not be limited to, four parameter logistic plots.

The invention will be further understood with reference to the following experimental examples.

### Materials and methods

### Preparation of calibration material

Pleural and ascites fluids were collected from cancer patients under informed consent using standard protocols. Typically fluids were collected by insertion of a drain into the chest cavity or peritoneal cavity under local anaesthetic. The drain might be inserted with or without image-guided control (eg Ultrasound) depending on local protocols and the practice of the treating clinician.
i) Pleural effusion should be collected into a sterile chest drain container in the standard manner for drainage of a pleural effusion.
ii) Ascites collected into a sterile drainage bag via a peritoneal drain in the standard manner for drainage of ascites

No chemicals need be added to the bag/container while the fluid is draining into it.

The bag/container should be collected either when full or on a daily basis whichever is sooner.

In Class II Hood, 1 Litre of fluid was transferred into 20 x 50ml sterile tubes using sterile 25 ml pipettes and centrifuged 400g for 5 minutes.

Supernatants were poured off into 2 sterile 500ml tissue culture flasks and Sodium Azide added to 0.01 % (1µl of 10% stock to 1 ml supernatant). Aprotinin (protease inhibitor) was added to 1µ/ml (1µ of 1mg/ml Aprotinin stock in PBS to 10ml of supernatant). Supernatants were then poured into non-sterile 50ml tubes and stored at -20ºC

### List of reagents:

Sodium Azide stock stored @ RT,
Aprotinin = Calbiochem 616370
Aprotinin stock stored in 50µl aliquots @ -20 °C
PBS = phosphate buffered saline

### Standard immunoassay for autoantibodies

The general immunoassay methodology is exemplified herein using recombinant tumour marker antigens but it will be appreciated that the same assay protocol may be adapted for use with other (auto)antigens.

Samples of tumour marker antigens were prepared by recombinant expression, following analogous methods to those described in WO 99/58978.

Briefly, cDNAs encoding the marker antigens of interest were cloned into the pET21 vector (Invitrogen) which has been modified to encode a biotin tag and a 6xhistidine tag to aid in purification of the expressed protein. The resulting clones were grown in a suitable bacterial host cell (in inclusion bodies), the bacteria lysed and denatured and the expressed antigens recovered via Nickel chelate affinity columns (Hi-trap, commercially available from Amersham, following manufacturer's protocol). The expressed antigens were renatured by dialysis in appropriate buffer and the yield of expressed protein assessed by SDS-PAGE, western blot and ELISA and quantitated prior to storage.

The negative control VOL is empty vector (i.e. no cloned cDNA) which still includes the His and biotin tag sequences.

GenBank accession numbers for a number of marker cDNAs are as follows:
P53: B003596
c-myc: V00568
ECD6 (HER2) extracellular domain: M11730
NY-ESO: NM_001327
BRCA2: U43746
BRCA1 delta 9-10: NM_007302
Annexin X1-A: NM_145868
PSA: NM_001648
CAGE: NM_182699 XM_291343
GBU4-5: NM_001110822 XM_001713629 XM_001713630 XM_001713631
Annexin 1: NM_000700
Annexin 2: NM_004039

1. Antigens and VOL (negative control) were diluted to appropriate concentrations in 0.1 M carbonate buffer. Antigen dilutions were dispensed at 50µl/well into the rows of a Falcon micotitre plate according to plate layout using an electronic multi-channel pipette. Plates were covered and stored at 4ºC for 48 h.
2. Plates were washed once in PBS + 0.1 % tween 20 using an automated plate washer then tapped dry on tissue paper.
3. Plates were blocked with high salt incubation buffer (HSBT, PBS + 0.5M NaCl + 0.1% casein + 0.1% Tween™ 20) at 200 µl/well for one hour or until required for use (store covered at 4ºC).
4. Test samples of patient bodily fluid and calibrator materials were diluted as appropriate in HSBT at room temp.
5. Plates were emptied and tapped dry on tissue paper. Each diluted test sample (or calibrator material) was dispensed at 50µl/well into all wells of the microtitre plate using an electronic multi-channel pipette. Plates were covered and incubated for 1.5 hour at room temp with shaking.
6. Wash step: Plates were washed three times in PBS + 0.1% tween 20 using an automated plate washer then tapped dry on tissue paper.
7. Horseradish peroxidase conjugated rabbit anti-human IgG&M (Jackson, 1/10,000 in HSBT) or rabbit anti-human IgG (Dako, 1/5000 in HSBT) was dispensed at 50 µl/well into all wells of the microtitre plate. HRP-conjugated rabbit anti-mouse Ig (1/1000 in HSBT) was used for assays employing mouse monoclonal antibodies. Plates were then incubated at room temp for 1 hour with shaking.
8. Plates were washed as inm step 6.
9. Pre-prepared TMB substrate was added at 50µl/well and plate incubated on bench for 1 min. Plates were gently tapped to mix.
10. Optical density of wells was determined at 650 nm using a standard plate reader protocol.

### Example 1 (comparative): Monoclonal antibodies as calibrators

Monoclonal antibodies were investigated as potential calibrator materials in autoantibody assays (data not shown). Although reproducible dilution responses could be produced, these could not be used as calibrator curves. It was considered that this was an inefficient calibration system because the monoclonal antibodies were murine in origin and therefore required a different secondary antibody reporter system to that used to detect human autoantibodies in serum. Thus with this approach one is effectively using two different measuring systems and day to day variation due to the secondary antibody system can not be detected or calibrated for by the mouse monoclonal system. In addition, the monoclonal response is so specific that it can not effectively mimic the polyclonal response exhibited by human autoantibodies. This may explain why monoclonal antibodies are not used as calibrator materials in benign autoimmune diseases such as systemic lupus erythamatosis and rheumatoid arthritis.

Since monoclonal antibodies had been discounted as possible calibrator materials, the inventors chose to investigate human pleural and ascites fluids as possible sources of calibrator materials for the reasons outlined above.

### Example 2: Demonstration of Antigen Specificity of Autoantibodies in Human Fluids

Patient fluids were screened in a standard autoantibody assay at a 1 in 100 dilution (in HSBT) to determine those that contained autoantibodies against a selected antigen (Table 1). Figure 1 a & b show examples of inhibition of binding of the autoantibodies in two different pleural fluids to two different antigens by their pre-incubation with that antigen in solution. Thus, the inventors have shown that the selected antigens measure autoantibodies which are specific for that particular tumour associated antigen.

As an additional demonstration of the specificity of autoantibodies in pleural fluids for tumour associated antigens, Western Blots were performed on the recombinant antigens used as capture agents in the autoantibody assay. These were carried out according to standard methodologies described in the literature and were probed with pleural fluids selected as calibrators. The results can be seen in Figure 2 in which the specificity of each fluid for a particular antigen is demonstrated by strong bands of the correct size corresponding to the antibody binding to the antigen with little or no evidence of binding to bands of contaminating material. In Figure 3, serum was used to probe similar Western Blots and in this case strong binding to baterial contaminants that are present in all of the recombinant antigens can be seen. The inventors have observed that 7/67 (90%) of serum samples tested contain antibodies to bacterial proteins however, of the 54 pleural fluids screened by Western Blotting, only 4 (7%) showed any evidence of such binding.

Despite the fact that patients had metastatic cancer, and therefore presumably the cancer had been present for some time, some fluid samples were shown to have autoantibodies to a limited number of antigens (Table 1).

**Table 1: Screening of pleural fluids for autoantibodies against seven different tumour associated antigens. Levels of measured autoantibody are arbitrarily designated low, intermediate or high.**

| | Numer of fluids in each calibrator group | | | | | | |
|---|---|---|---|---|---|---|---|
| | P53 | C-myc | ECD6 | NYESO | BRCA2 | PSA | Annexin Xla |
| low | 61 | 53 | 62 | 59 | 61 | 21 | 26 |
| Intermediate | 1 | 9 | 1 | 1 | 2 | 5 | 3 |
| High | 3 | 3 | 2 | 5 | 2 | 5 | 2 |

In benign autoimmune diseases the auto-antigens are usually much more limited for each particular disorder. In this respect the data on the bodily fluids in cancer patients reported by the inventors are different. The presence of different autoantibodies in different cancer patients makes developing an overall calibration system for a multiple autoantibody test much more challenging and complex. Following screening of these fluids those positive against an antigen were investigated further for their best use as a calibrator for the assay.

### Example 3: Antigen and VOL titrated and fluids titrated

The possibility of using patient fluids as a calibration system was initially investigated using a double titration system, in which assay plates were coated with titrations of both antigen and VOL (see Table 2a). Antigens and VOL were allowed to adsorb to the plate for a minimum of 48 hours after this time the plate was washed and blocked for 90 min with PBS containing casein (0.1 % w/v), NaCl (0.5M) and Tween 20 (0.1% w/v). During the blocking incubation a set of patient fluid calibrator titrations (in HSBT) were prepared in tubes. Following removal of the blocking buffer, these were added to the empty plate as shown in Table 2b and incubated for 90 min. The remainder of the assay was performed as described in materials and methods.

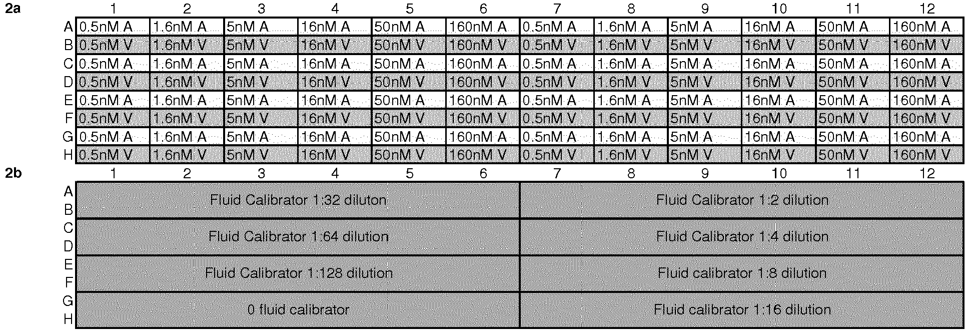

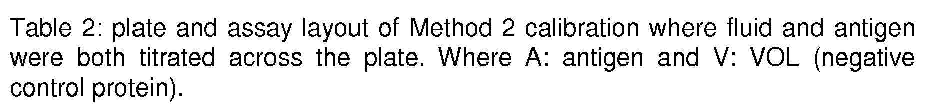

By analysing the data by this method a large amount of data was produced, not all of it applicable for the function of a calibration system. Figure 4 is an example of a result generated by this assay format using pleural fluid as the calibration material.

By using this method it was demonstrated that patients' fluids could produce effective titration curves at different fluid and/or antigen dilutions. This method produced a large amount of data, but did not appear to optimise the use of such fluids as assay calibrators. As is demonstrated clearly in Figure 5, binding of fluids to low antigen concentrations is not useful in calibration due to low signal and narrow dynamic range. However at high static concentration of antigen such as 160 and 50nM there is a broad dynamic range of OD values derived from binding of the patient fluid to the antigen; giving rise to scope for calibration of a wide range of autoantibody measurements.

This result led to calibrator Method 3 where the inventors utilised this observation so that plates were coated with a static concentration of antigen and the patient fluid titrated as a calibrator.

### Example 4: Antigen and VOL at a static concentration and fluids titrated

The inventors found this method produced the most useful data sets in relation to the autoantibody assays because it reduced the amount of data being collected to a level that could easily be produced reproducibly and analysed efficiently. By reducing the amount of wells being assayed per calibration run, it was also possible to run control samples concurrently on the same plates as the calibrator curves. It had also been demonstrated that serum autoantibody measurements at 160 and 50nM gave the most useful information and that calibration curves measured against these two antigen concentrations provided the greatest dynamic range for calibration. It was therefore decided to investigate this method in multiple settings.

Initial experiments were conducted to determine the reproducibility of patient fluids against seven different antigens. These assays were performed on plates coated with antigen at 160nM and 50nM. Antigens were allowed to adsorb to the plate for a minimum of 48 hours after this time the plate was washed and blocked for 90 min with HSBT. During the blocking incubation a set of multiple patient fluid calibrator titrations were prepared in tubes. Following removal of the blocking buffer, these were added to the empty plate and incubated for 90 min. The remainder of the assay was performed as described in materials and methods.

To each plate, calibrator fluid was applied in duplicate down a doubling dilution range starting at 1:2. This assay was performed on 10 occasions to determine the reproducibility of the signal. The results in Figure 5 show representative graphs of the mean shape of the curves produced from the 10 runs. The inter-assay variation is represented in the form of error bars which are shown in the form of standard deviations associated with the mean of the 10 runs. The inter-assay coefficient of variation (CVs) for the reaction with each antigen are shown in Table 3.

**Table 3: Inter-assay CVs (%) for each drainage fluid dilution reacting with a range of tumour-associated antigens. Figures are calculated from calculated from ten runs.**

| CV of Raw OD Data | | | | | | | |
|---|---|---|---|---|---|---|---|
| | C-myc | P53 | PSA | Annexin | BRCA₂ | ECD6 | NYESO |
| dilution 1 | 11 | 9 | 8 | 6 | 13 | 13 | 7 |
| dilution 2 | 9 | 11 | 10 | 6 | 10 | 21 | 8 |
| dilution 3 | 11 | 14 | 12 | 8 | 9 | 20 | 14 |
| dilution 4 | 14 | 17 | 16 | 11 | 11 | 26 | 18 |
| dilution 5 | 15 | 13 | 17 | 17 | 13 | 26 | 21 |
| dilution 6 | 17 | 12 | 19 | 18 | 14 | 25 | 25 |
| dilution 7 | 16 | 10 | 17 | 21 | 17 | 20 | 20 |
| dilution 8 | 17 | 10 | 12 | 19 | 15 | 17 | 17 |

Further development of the above experiments led to the following calibrator protocol. However it should be noted that this method is given as an example but is not the only method in which bodily fluids such as ascites and pleural effusions might be used to produce a calibration system. The described method allows the use of one patient fluid as a calibrator that is serially diluted down a plate coated with a static concentration of antigen (for example either 50nM or 160nM of antigen and VOL). It also allows the incorporation of control serum into the assay format. This method then plots the log fluid dilution against the log OD of the fluid calibrators to produce a 4 parameter logistic curve. This curve was then used to extrapolate the equivalent calibrator fluid dilution value from the log optical density values of the control serum.

### Example 5: optimisation of the calibration method:

The following is an example of an assay plan to calibrate anti-tumour marker autoantibody assays.

96 well microtitre plates were coated at both 160nM and 50nM levels of antigen (Annexin Xla, PSA, p53, ECD6, BRCA2, NYESO, and c-myc) and the negative control protein, VOL as displayed in Table 4. The antigens were allowed to adsorb to the plate for at least 48 hours at 4ºC. After this time the plate was washed and blocked for 90 min with HSBT. During blocking incubation the calibrator dilutions and control sera (diluted 1:100 in HSBT) were prepared.

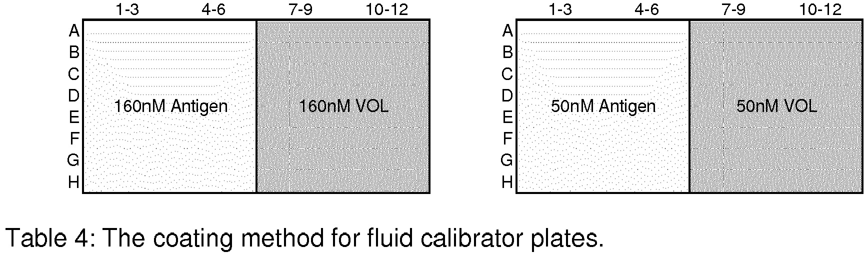

Following removal of the blocking buffer, the calibrator fluid and control sera were added to the empty plate as shown in Table 5 and incubated for 90 min. The remainder of the assay was performed as described in materials and methods. Antigen titration curves were constructed using mean values of triplicates for each calibrator. The log of the calibrator fluid dilution and the log of the mean optical density were plotted in a graph and used to plot a 4 parameter logistic curve to fit the data. This curve was then used to extrapolate the equivalent calibrator fluid dilution value from the log optical density values of the control serum.

For each day that autoantibody assays are run one set of calibrator plates are also run. Using seven antigens at both the 50nM and 160nM concentration a total of 14 calibration plates are required.

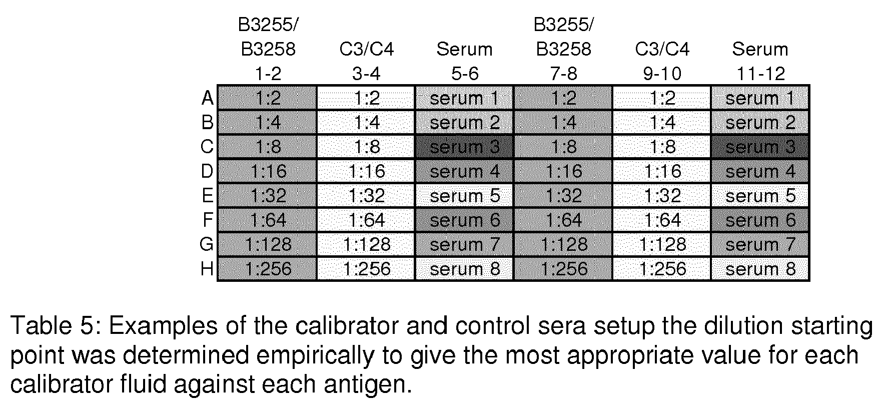

These experiments were initially performed using two different calibrator fluids, each of which consisted of a pool of two fluids taken from the same patient but at different times and 8 serum controls. The data from five assay runs are displayed in Figures 6-12.

The next experiment focussed on the selection of one fluid calibrator for each antigen. The principle characteristic which defines a good calibrator fluid is good dynamic range. If a log/log plot is used, then other useful characteristics are:
- Linearity of log/log plot.
- Suitability of slope of log/log plot
- Reproducibility of slope of log/log plot.

### Example 6: Effect of calibration on day to day variability in control samples

Control samples were run on the same plate as the calibrator curves to investigate whether by using the pleural calibration curve to extrapolate back to a log dilution value, we could correct for day to day variation observed in the control samples. The data showing the variation in raw OD values compared with the values extrapolated from the calibrator curves is shown in Figures 13-19. It can be seen from these figures that for most antigens, extrapolation from the log/log plot of the calibration line improves the day to day reproducibility of the measurement of autoantibody levels in serum.

### Example 7: Comparison of serum with pleural fluids as calibrator materials

Assays to measure autoantibodies in autoimmune diseases have used serum or plasma as calibrator materials. Drainage fluids have a number of advantages over blood products. They are available in very large volumes, are stable under storage at low temperatures for long periods of time and can therefore be used to provide reproducible calibrator materials for many assays. The collection of a large volume at a single timepoint has potentially important advantages over multiple sequential collection of much smaller volumes of serum. Firstly, metastatic disease is an incurable condition and patients will all eventually die of their disease making sequential blood sampling very difficult and eventually impossible. Secondly, the titre of autoantibodies might change with time and so sequential blood samples might not be comparable. Thirdly, with antigenic drift the humoral immune response might change to another immuno-dominant antigen(s). Even if blood samples were taken from primary breast cancer patients (i.e. at an earlier stage) then if a patient is cured by their treatment the autoantibody response may decrease and not be detected in sequential samples. All of the above means that the use of bodily fluids as described in this application are believed to be novel and inventive. In order to assess other advantages of using fluids a direct comparison with matched serum samples was performed. Dilution series of serum and drainage fluids taken from the same patient were assayed for their ability to bind to a range of tumour associated antigens. The results are shown in Figures 20 and 21.

It can be seen in Figure 20 that the pattern of reactivity of the fluid and serum were similar across a range of antigens. However Figure 21 shows that for the antigens which show positive reactivity for autoantibodies (i.e. ECD6, PSA & Annexin XI-a) the signal in serum is generally lower than the signal in the pleural fluid. In addition, the pattern of reactivity differs with the serum autoantibodies having a much lower level of reactivity with PSA relative to ECD6. This would suggest that although samples C7 and 11828 are from the same patient, the pleural drainage fluid C7 would provide a better calibrator for PSA autoantibodies due to its greater dynamic range. This particular finding was extremely unexpected.

### Example 8: Use of pleural fluids to calibrate assays in the clinical laboratory setting

In order to validate the use of pleural fluids under the conditions encountered in a high throughput laboratory performing autoantibody assays the following experiments were run:

### Calibration:

After first identifying suitable calibrator fluids with specificity for each antigen (see Example 2) and optimising the dilution range to span the dynamic range of the assay (see Example 4), calibrator plates were coated with antigen as in Table 6:

**Table 6: Format of antigen coated plates to be used for calibration.**

| | 1 | 2 | 3 | 4 | 5 | 6 | 7 | 8 | | 10 | 11 | 12 | Example of Fluid Dilution Series: |
|---|---|---|---|---|---|---|---|---|---|---|---|---|---|
| A | | | | | | | | | | | | | 1 in 8 |
| B | | | | | | | | | | | | | 1 in 16 |
| C | Antigen at | | | VOL at | | | Antigen at | | | VOL at | | | 1 in 32 |
| D | 160 nM | | | 160 nM | | | 50 nM | | | 50 nM | | | 1 in 64 |
| E | | | | | | | | | | | | | 1 in 128 |
| F | | | | | | | | | | | | | 1 in 256 |
| G | | | | | | | | | | | | | 1 in 512 |
| H | | | | | | | | | | | | | 1 in 1024 |

Calibrator fluids specific for each of the antigens in the panel shown in Figure 3 were serially diluted over the appropriate range and added to the plate above as shown in the example. These plates were assayed according to the standard protocol.

### Serum Samples:

Plates were coated with antigen as in Table 7 and used to assay a number of different serum samples that had previously been shown to have antigen-specific autoantibody levels. Assays were performed according to the standard protocol.

**Table 7: Format of plates used to assay serum samples to test the performance of the calibration system under clinical laboratory conditions.**

| | 1 | 2 | 3 | 4 | 5 | 6 | 7 | 8 | 9 | 10 | 11 | 12 |
|---|---|---|---|---|---|---|---|---|---|---|---|---|
| | 0n M | 1.6nM | 5nM | 16nM | 50 nM | 160nM | 0nM | 1.6nM | 5nM | 16nM | 50 nM | 160nM |
| A | | GBU4-5 | | | | | | p53 | | | | |
| B | | VOL | | | | | | c-myc | | | | |
| C | | Annexin I | | | | | CAGE | CAGE | | | | |
| D | | Annexin II | | | | | | NY-ESO-1 | | | | |
| E | | p53 | | | | | | GBU4-5 | | | | |
| F | | c-myc | | | | | | VOL | | | | |
| G | | CAGE | | | | | | Annexin I | | | | |
| H | | NY-ESO-1 | | | | | | Annexin II | | | | |

The assays described above were performed twice per day (morning and afternoon) for 6 days over a 2-week time span (runs 1 to 12).

### Variability Runs:

In order to test the calibration system, variability had to be introduced into the assay output. This was achieved by reducing the concentration of horseradish peroxidase labelled secondary antibody in order to produce a lower signal on all plates. This was performed twice on the seventh assay day (runs 13 and 14).

### Calibration of serum samples:

Four-parameter logistic (4pl) plots were constructed for each set of calibrator data. For each plot the bottom asymptote was set to zero and the slope was set at 1. The top asymptote was constrained with a maximum of 2. The data from each individual curve was solved to minimise the sum of squared residuals. This provided values for the four parameters (top asymptote, bottom asymptote, slope and EC₅₀) which were then used in a formula to read serum samples from the calibrator curve. In Figure 22 the 4pl plot of optical density versus log calibrator fluid dilution for runs 1 to 12 is shown (as solid grey line) along with error bars representing the standard deviation for the data set. The second curve on each Figure (broken line) is the mean 4pl plot of the variability runs (runs 13 and 14) with standard deviations as error bars.

The equations describing the plots shown in Figure 22 were used to correct each serum sample according to its respective calibrator. This resulted in the value being expressed as an arbitrary unit corresponding to the log dilution of the calibrator fluid (RU values). The effect that this had on variation between runs is shown in Figure 23 for a number of different serum samples where the uncalibrated values are shown as open triangles and the values corrected according to the calibration curve for that run are shown as solid circles. The broken lines represent mean of the calibrated values plus or minus 3 standard deviations. Note how the variability introduced in runs 13 and 14 is reduced significantly by calibration.

### Example 9: Storage of Frozen Calibrator Series

Since the dilution of serial titrations is time consuming and prone to reproducibility errors we investigated the differences between a set of 'frozen' calibrators; where stocks of calibrator pleural fluid dilutions were prepared, aliquotted and frozen at - 20C; and those freshly prepared on the day. The results of this study can be seen in Figures 24-30. It can be seen that there was very little difference between calibrator series that had been prepared freshly each day and those that had been made up in bulk quantities and frozen in aliquots. This would suggest that, when being used as calibrator materials, diluted patient fluids are stable to storage at low temperature (-20°C) and can be stored for up to extended periods without loss in activity. This is therefore a valid method for reducing inter-run variation.

### Example 10: Calibration Using Fluids Derived from Patients with Cancers Other Than Breast Cancer

Some tumour associated antigens and the autoantibodies they elicit are not tumour type specific. Therefore it is possible that fluids derived from patients with lung cancer for example, could be used to calibrate autoantibody assays for the early diagnosis of breast cancer and vice versa. In order to test this theory, pleural fluids taken from patients with colon, ovarian, lung, liver and pancreatic cancer were screened against a panel of tumour associated antigens. Once positivity had been established, calibration dilution curves were prepared and tested against the antigens. The experiment was repeated 5 times on separate days to assess reproducibility. In Figure 31 it can be seen that autoantibody binding to a range of different tumour associated antigens can be detected in fluids from patients with colon cancer (a and e), ovarian cancer (b), lung cancer (c), liver cancer (d and f) and pancreatic cancer (g). These responses appear to be reproducible and titrate out as the fluid is diluting indicating that they have potential to be used as calibrator materials in autoantibody assays for the early diagnosis of breast and other cancers.

### Example 11: Use of Human Fluids for Quantification of the Amount of Protein on Solid Surfaces

Passive adsorption of proteins to plastic surfaces such as to the wells of microtitre plate is not clearly defined and controlled and can depend on factors such as the unevenness of the surface and charges on the protein and plastic. Hence it would be useful to be able to quantify how much protein has adsorbed and to be able to relate this to other proteins and other surfaces. Colourimetric protein assays are too insensitive for such measurements. Adsorption isotherm methodologies have been described (Kelso et al) but these rely on the availability of a labelled tracer molecule. Antibodies against protein tags (such as the His-tag) can also be used but they are usually murine in origin and so rely on a different reporter system to that used for the measurement of human autoantibodies.

During screening of human fluids against a range of tumour associated antigens two of the fluids were observed to bind to all proteins including the negative control, VOL (Figure 32). VOL is a recombinant peptide cloned and expressed in exactly the same manner as the antigens but just consisting of a biotin tag sequence and a his-tag. Therefore the human antibodies within these fluids must be binding to one or both of these tags. Since the tags are also present on all of the recombinant tumour associated antigens, the fluid could be used to quantitate the amount of protein adsorbed to the wells of the plate. Table 6 shows the ratio of signal at 50nM to signal at 160nM for VOL at each concentration. It can be seen that the ratio of binding signal to VOL at 50nM and 160nM is relatively constant across all dilutions of pleural fluid. This would suggest that the signal measured is related to amount of protein on the plate and so this system can be used to quantify protein levels.

**Table 8: Measurement of autoantibody binding to VOL of drainage fluid 16. Ratio of signal at 50nM to signal at 160nM for VOL at each fluid dilution.**

| Fluid Dilution | Run 1 | Run 2 | Run 3 | Run 4 | Run 5 |
|---|---|---|---|---|---|
| 1 in 8 | 0.65 | 0.58 | 0.53 | 0.76 | 0.62 |
| 1 in 16 | 0.54 | 0.52 | 0.42 | 0.87 | 0.92 |
| 1 in 32 | 0.51 | 0.5 | 0.41 | 0.82 | 0.71 |
| 1 in 64 | 0.54 | 0.58 | 0.51 | 0.84 | 0.72 |
| 1 in 128 | 0.69 | 0.64 | 0.53 | 1.03 | 0.76 |

### Example 12: Detection and calibration of non-specific binding

Non-specific binding is a problem inherent in any serological immuno-assay due to the high concentrations of immunoglobulins present in serum which tend to bind non-specifically to the plastic of the plate or the coating antigen. Non-specific binding signals vary from serum sample to serum sample but can be so high that they mask the specific reaction of the analyte.

In the previous section fluids that bound to VOL were identified. The reaction of serum antibodies is non-specific in that it is not directed against any particular antigen. These pleural fluids may therefore be used to detect and correct for non-specific binding.

### Example 13: Use of ascites fluids as a calibrator material

In addition to pleural fluids, ascites fluids were effective as an analogous calibration system to that used in the previous examples. These assays were performed on plates coated with antigen at 160nM and 50nM. Antigens were allowed to adsorb to the plate for a minimum of 48 hours, after this time the plate was washed and blocked for 90 min with HSBT. During the blocking incubation a set of multiple patient ascites fluid calibrator titrations were prepared in tubes. Following
removal of the blocking buffer, these were added to the empty plate and incubated for 90 min. The remainder of the assay was performed as described in Materials and Methods.

To each plate ascites fluid was applied in duplicate down a doubling dilution range starting at 1:2. This assay was performed on 4 occasions to determine the reproducibility of the signal. The results in Figures 33-35 show representative graphs of the mean shape of the VOL corrected curves produced from the 4 runs. The inter-assay variation is represented in the form of error bars which are shown in the form of standard deviations associated with the mean of the 4 runs.

## Claims

1. Use of a calibrator material comprising mammalian bodily fluid to calibrate an immunoassay for detection of autoantibodies, wherein said calibrator material comprises a drainage fluid, exudate or transudate.

2. Use according to claim 1 wherein the calibrator material
a) comprises human bodily fluid, or
b) comprises bodily fluid collected from a body cavity or space in which a tumour is or was present or with which a tumour is or was associated.

3. Use according to claim 2 wherein the calibration material comprises
a) pleural fluid collected from one or more subjects with cancer, or
b) ascites fluid collected from one or more subjects with cancer.

4. Use according to any one of claims 1 to 3 wherein the immunoassay is for
a) detection of autoantibodies to cancer, or
b) for detection of autoantibodies to early neoplastic change.

5. A method of calibrating an immunoassay for detection of autoantibodies which comprises:
(a) contacting each of a plurality of different dilutions of a calibration material comprising a mammalian bodily fluid with an antigen specific for an autoantibody, wherein said mammalian bodily fluid is known to contain autoantibodies immunologically specific for the antigen and wherein said calibrator material comprises a drainage fluid, exudate or transudate;
(b) detecting the amount of specific binding between said antigen and autoantibody present in the calibration material; and
(c) plotting or calculating a curve of the amount of said specific binding versus the dilution of the calibration material for each dilution of calibration material used in step (a), thereby calibrating an immunoassay using said antigen for detection of said autoantibody.

6. The method of claim 5 wherein the calibration material
a) comprises human bodily fluid,
b) comprises bodily fluid collected from one or more subjects with cancer, or
c) comprises bodily fluid collected from a body cavity or space in which a tumour is or was present or with which a tumour is or was associated.

7. The method of claim 6 wherein the calibration material comprises
a) pleural fluid collected from one or more subjects with cancer, or
b) ascites fluid collected from one or more subjects with cancer.

8. The method of any one of claims 5 to 7 wherein the calibration material comprises human bodily fluid which is known to contain native human autoantibodies which are immunologically specific for a tumour marker protein.

9. The method of any one of claims 5 to 8 wherein the antigen of part (a) is used at a concentration
a) greater than 20nM,
b) in the range of from 20nM to 180nM, or
c) in the range of from 50nM to 160nM.

10. An immunoassay kit for detection of autoantibodies, said kit comprising a set of calibration standards for use in calibrating an immunoassay for detection of autoantibodies, wherein each calibration standard in said set comprises a different dilution of a mammalian bodily fluid, said mammalian bodily fluid being known to contain native human autoantibodies, and wherein the mammalian bodily fluid is a drainage fluid, exudate or transudate and an immunoassay reagent comprising an antigen immunologically specific for said autoantibodies.

11. An immunoassay kit as claimed in claim 10 wherein said mammalian bodily fluid
a) is a human bodily fluid,
b) is a fluid collected from one or more subjects with cancer, or
c) is bodily fluid collected from a body cavity or space in which a tumour is or was present or with which a tumour is or was associated.

12. An immunoassay kit as claimed in claim 11 wherein the mammalian bodily fluid comprises
a) pleural fluid collected from one or more subjects with cancer, or
b) ascites fluid collected from one or more subjects with cancer.

13. An immunoassay kit as claimed in any one of claims 10 to 12 wherein the mammalian bodily fluid is human bodily fluid which is known to contain native human autoantibodies immunologically specific for a tumour marker protein.

14. Use according to claim 1 or 2 wherein the immunoassay is for detection of autoantibodies to benign autoimmune disease.

## Patentansprüche

1. Verwendung eines Kalibratormaterials, umfassend ein Säugetierkörperfluid, um einen Immuntest zur Erfassung von Autoantikörpern zu kalibrieren, wobei das Kalibratormaterial ein Drainagefluid, ein Exsudat oder ein Transsudat umfasst.

2. Verwendung nach Anspruch 1, wobei das Kalibratormaterial
a) ein menschliches Körperfluid umfasst, oder
b) ein Körperfluid umfasst, welches von einem Körperhohlraum oder - raum gesammelt worden ist, in welchem ein Tumor vorhanden ist oder vorhanden war, oder welchem ein Tumor zugehörig ist oder zugehörig war.

3. Verwendung nach Anspruch 2, wobei das Kalibrierungsmaterial
a) Pleurafluid, welches von einem oder mehreren Probanden mit Krebs gesammelt worden ist, oder
b) Aszitesfluid, welches von einem oder mehreren Probanden mit Krebs gesammelt worden ist, umfasst.

4. Verwendung nach einem der Ansprüche 1 bis 3, wobei der Immuntest für
a) eine Erfassung von Autoantikörpern gegen Krebs, oder
b) zur Erfassung von Autoantikörpern gegen eine frühe neoplastische Veränderung ist.

5. Verfahren zum Kalibrieren eines Immuntests zur Erfassung von Autoantikörpern, welches umfasst:
(a) Kontaktieren jeder von einer Mehrzahl von verschiedenen Dilutionen eines Kalibrierungsmaterials, umfassend ein Säugetierkörperfluid mit einem für einen Autoantikörper spezifischen Antigen, wobei das Säugetierkörperfluid bekannt ist, für das Antigen immunologisch spezifische Autoantikörper zu enthalten, und wobei das Kalibratormaterial ein Drainagefluid, ein Exsudat oder ein Transsudat umfasst;
(b) Erfassen der Menge von spezifischer Bindung zwischen dem Antigen und dem Autoantikörper, welche in dem Kalibrierungsmaterial vorhanden ist; und
(c) Aufzeichnen oder Berechnen einer Kurve der Menge von spezifischer Bindung gegenüber der Dilution des Kalibrierungsmaterials für jede Dilution von Kalibrierungsmaterial, welche in Schritt (a) verwendet wird, so dass dadurch ein Immuntest unter Verwendung des Antigens zur Erfassung des Autoantikörpers kalibriert wird.

6. Verfahren nach Anspruch 5, wobei das Kalibrierungsmaterial
a) ein menschliches Körperfluid umfasst,
b) ein Körperfluid umfasst, welches von einem oder mehreren Probanden mit Krebs gesammelt worden ist, oder
c) ein Körperfluid umfasst, welches von einem Körperhohlraum oder - raum gesammelt worden ist, in welchem ein Tumor vorhanden ist oder vorhanden war, oder welchem ein Tumor zugehörig ist oder zugehörig war.

7. Verfahren nach Anspruch 6, wobei das Kalibrierungsmaterial
a) Pleurafluid, welches von einem oder mehreren Probanden mit Krebs gesammelt worden ist, oder
b) Aszitesfluid, welches von einem oder mehreren Probanden mit Krebs gesammelt worden ist, umfasst.

8. Verfahren nach einem der Ansprüche 5 bis 7, wobei das Kalibrierungsmaterial ein menschliches Körperfluid umfasst, welches bekannt ist, für ein Tumormarkerprotein immunologisch spezifische native menschliche Autoantikörper zu enthalten.

9. Verfahren nach einem der Ansprüche 5 bis 8, wobei das Antigen aus Teil (a) bei einer Konzentration von
a) mehr als 20 nM,
b) in dem Bereich von 20 nM bis 180 nM, oder
c) in dem Bereich von 50 nM bis 160 nM verwendet wird.

10. Immuntestsystem zur Erfassung von Autoantikörpern, wobei das System einen Satz von Kalibrierungsstandards zur Verwendung in einer Kalibrierung eines Immuntests für eine Erfassung von Autoantikörpern umfasst, wobei jeder Kalibrierungsstandard in dem Satz eine unterschiedliche Dilution eines Säugetierkörperfluids umfasst, wobei das Säugetierkörperfluid bekannt ist, native menschliche Autoantikörper zu enthalten, und wobei das Säugetierkörperfluid ein Drainagefluid, ein Exsudat oder ein Transsudat ist und ein Immuntest-Reagens ein für die Autoantikörper immunologisch spezifisches Antigen umfasst.

11. Immuntestsystem nach Anspruch 10, wobei das Säugetierkörperfluid
a) ein menschliches Körperfluid ist,
b) ein Fluid ist, welches von einem oder mehreren Probanden mit Krebs gesammelt worden ist, oder
c) ein Körperfluid ist, welches von einem Körperhohlraum oder -raum gesammelt worden ist, in welchem ein Tumor vorhanden ist oder vorhanden war, oder welchem ein Tumor zugehörig ist oder zugehörig war.

12. Immuntestsystem nach Anspruch 11, wobei das Säugetierkörperfluid
a) Pleurafluid, welches von einem oder mehreren Probanden mit Krebs gesammelt worden ist, oder
b) Aszitesfluid, welches von einem oder mehreren Probanden mit Krebs gesammelt worden ist, umfasst.

13. Immuntestsystem nach einem der Ansprüche 10 bis 12, wobei das Säugetierkörperfluid ein menschliches Körperfluid ist, welches bekannt ist, für ein Tumormarkerprotein immunologisch spezifische native menschliche Autoantikörper zu enthalten.

14. Verwendung nach Anspruch 1 oder 2, wobei der Immuntest für eine Erfassung von Autoantikörpern gegen eine gutartige Autoimmunerkrankung ist.

## Revendications

1. Utilisation d'une substance d'étalonnage comprenant un fluide corporel mammalien pour étalonner un immunodosage destiné à détecter des auto-anticorps, dans laquelle ladite substance d'étalonnage comprend un fluide de drainage, un exsudat ou un transsudat.

2. Utilisation selon la revendication 1 dans laquelle la substance d'étalonnage
a) comprend un fluide corporel humain, ou
b) comprend un fluide corporel recueilli à partir d'une cavité ou d'un espace corporel dans lequel une tumeur est ou a été présente ou auquel une tumeur est ou a été associée.

3. Utilisation selon la revendication 2 dans laquelle la substance d'étalonnage comprend
a) un liquide pleural recueilli auprès d'un ou de plusieurs sujets atteints de cancer, ou
b) un liquide d'ascite recueilli auprès d'un ou de plusieurs sujets atteints de cancer.

4. Utilisation selon l'une quelconque des revendications 1 à 3 dans laquelle l'immunodosage est destiné à
a) détecter des auto-anticorps dirigés contre le cancer ; ou
b) détecter des auto-anticorps dirigés contre une variation néoplasique précoce.

5. Procédé d'étalonnage d'un immunodosage destiné à détecter des auto-anticorps qui comprend :
(a) la mise en contact de chacune d'une pluralité de dilutions différentes d'une substance d'étalonnage comprenant un fluide corporel mammalien avec un antigène spécifique d'un auto-anticorps, dans lequel ledit fluide corporel mammalien est connu pour contenir des auto-anticorps immunologiquement spécifiques de l'antigène et dans lequel ladite substance d'étalonnage comprend un fluide de drainage, un exsudat ou un transsudat ;
(b) la détection de la quantité de liaison spécifique entre ledit antigène et l'auto-anticorps présent dans la substance d'étalonnage ; et
(c) la représentation graphique ou le calcul d'une courbe de la quantité de ladite liaison spécifique en fonction de la dilution de la substance d'étalonnage pour chaque dilution de substance d'étalonnage utilisée à l'étape (a), pour étalonner ainsi un immunodosage utilisant ledit antigène pour la détection dudit auto-anticorps.

6. Procédé selon la revendication 5 dans lequel la substance d'étalonnage
a) comprend un fluide corporel humain,
b) comprend un fluide corporel recueilli auprès d'un ou de plusieurs sujets atteints de cancer, ou
c) comprend un fluide corporel recueilli à partir d'une cavité ou d'un espace corporel dans lequel une tumeur est ou a été présente ou auquel une tumeur est ou a été associée.

7. Procédé selon la revendication 6 dans lequel la substance d'étalonnage comprend
a) un liquide pleural recueilli auprès d'un ou de plusieurs sujets atteints de cancer, ou
b) un liquide d'ascite recueilli auprès d'un ou de plusieurs sujets atteints de cancer.

8. Procédé selon l'une quelconque des revendications 5 à 7 dans lequel la substance d'étalonnage comprend un fluide corporel humain qui est connu pour contenir des auto-anticorps humains natifs qui sont immunologiquement spécifiques d'un marqueur protéique de tumeur.

9. Procédé selon l'une quelconque des revendications 5 à 8 dans lequel l'antigène de la partie (a) est utilisé à une concentration
a) supérieure à 20 nM,
b) dans la plage de 20 à 180 nM, ou
c) dans la plage de 50 à 160 nM.

10. Kit d'immunodosage pour la détection d'auto-anticorps, ledit kit comprenant un ensemble de références d'étalonnage pouvant être utilisées pour étalonner un immunodosage destiné à détecter des auto-anticorps, dans lequel chaque référence d'étalonnage dans ledit ensemble comprend une dilution différente d'un fluide corporel mammalien, ledit fluide corporel mammalien étant connu pour contenir des auto-anticorps humains natifs, et dans lequel le fluide corporel mammalien est un fluide de drainage, un exsudat ou un transsudat et un réactif d'immunodosage comprenant un antigène immunologiquement spécifique desdits auto-anticorps.

11. Kit d'immunodosage selon la revendication 10 dans lequel ledit fluide corporel mammalien
a) est un fluide corporel humain,
b) est un fluide recueilli auprès d'un ou de plusieurs sujets atteints de cancer, ou
c) est un fluide recueilli à partir d'une cavité ou d'un espace corporel dans lequel une tumeur est ou a été présente ou auquel une tumeur est ou a été associée.

12. Kit d'immunodosage selon la revendication 11 dans lequel le fluide corporel mammalien comprend
a) un liquide pleural recueilli auprès d'un ou de plusieurs sujets atteints de cancer, ou
b) un liquide d'ascite recueilli auprès d'un ou de plusieurs sujets atteints de cancer.

13. Kit d'immunodosage selon l'une quelconque des revendications 10 à 12 dans lequel le fluide corporel mammalien est un fluide corporel humain qui est connu pour contenir des auto-anticorps humains natifs immunologiquement spécifiques d'un marqueur protéique de tumeur.

14. Utilisation selon la revendication 1 ou 2 dans laquelle l'immunodosage est destiné à détecter des auto-anticorps dirigés contre une maladie auto-immune bénigne.
